(19) Europäisches Patentamt
European Patent Office
Office européen des brevets

(11) Veröffentlichungsnummer: **0 301 393 B1**

(12) **EUROPÄISCHE PATENTSCHRIFT**

(45) Veröffentlichungstag der Patentschrift: **14.08.91**

(51) Int. Cl.[5]: **C07D 249/08**, C07D 405/06, C07D 233/60, A01N 43/50, A01N 43/653, //C07C49/255

(21) Anmeldenummer: **88111667.7**

(22) Anmeldetag: **20.07.88**

Die Akte enthält technische Angaben, die nach dem Eingang der Anmeldung eingereicht wurden und die nicht in dieser Patentschrift enthalten sind.

# (54) 1,4-Disubstituierte 1-Azolyl-3,3-dimethylbutan-Derivate.

(30) Priorität: **31.07.87 DE 3725397**

(43) Veröffentlichungstag der Anmeldung: **01.02.89 Patentblatt 89/05**

(45) Bekanntmachung des Hinweises auf die Patenterteilung: **14.08.91 Patentblatt 91/33**

(84) Benannte Vertragsstaaten: **BE CH DE FR GB IT LI NL**

(56) Entgegenhaltungen:
**EP-A- 0 054 865**
**EP-A- 0 055 833**
**EP-A- 0 100 952**
**EP-A- 0 175 233**
**EP-A- 0 226 916**

(73) Patentinhaber: **BAYER AG**
**W-5090 Leverkusen 1 Bayerwerk(DE)**

(72) Erfinder: **Elbe, Hans-Ludwig, Dr.**
**Dasnöckel 59**
**W-5600 Wuppertal 11(DE)**
Erfinder: **Marhold, Albrecht, Dr.**
**Carl-Duisberg-Strasse 329**
**W-5090 Leverkusen(DE)**
Erfinder: **Büchel, Karl Heinz, Prof. Dr.**
**Dabringhauser Strasse 42**
**W-5093 Burscheid(DE)**
Erfinder: **Dutzman, Stefan, Dr.**
**Leinenweberweg 33**
**W-4000 Düsseldorf 13(DE)**
Erfinder: **Reinecke, Paul, Dr.**
**Steinstrasse 8**
**W-5090 Leverkusen 3(DE)**
Erfinder: **Brandes, Wilhelm, Dr.**
**Eichendorffstrasse 3**
**W-5653 Leichlingen(DE)**
Erfinder: **Hänssler, Gerd, Dr.**
**Am Arenzberg 58a**
**W-5090 Leverkusen 3(DE)**

**EP 0 301 393 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 1,4-disubstituierte 1-Azolyl-3,3-dimethylbutan-Derivate, mehrere Verfahren zu ihrer Herstellung sowie ihre Verwendung als Fungizide.

Es ist bereits bekannt geworden, daß zahlreiche Azolylcarbonyl-und -carbinol-Derivate fungizide Eigenschaften besitzen (vgl. US-A 4 549 900, EP-A 0 054 865 und EP-A 0 055 833 und DE-A 35 44 731). So lassen sich zum Beispiel 1-(4-Chlorphenyl)-2,2-dimethyl-4-(1,2,4-triazol-1-yl)-hexan-3-on; 1-(4-Chlorphenyl)-2,2-dimethyl-4-(1,2,4-triazol-1-yl)-hexan-3-ol; 1-(3-Brom-4-fluorphenyl)-2,2-dimethyl-4-(1,2,4-triazol-1-yl)-oct-7-en-3-ol und 1-(4-Methylphenyl)-2,2-dimethyl-4-(1,2,4-triazol-1-yl)-pentan-3-ol zur Bekämpfung von Pilzen einsetzen. Die Wirkung dieser Verbindungen ist jedoch in bestimmten Indikationsbereichen, insbesondere bei niedrigen Aufwandmengen und -konzentrationen, nicht immer ganz befriedigend.

Es wurden nun neue 1,4-disubstituierte 1-Azolyl-3,3-di-methylbutan-Derivate der Formel (I)

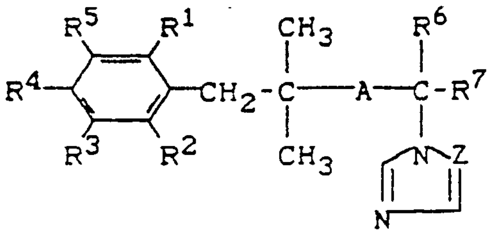

(I)

in welcher

| | |
|---|---|
| $R^1$ | für Wasserstoff, Fluor, Chlor oder Brom steht; |
| $R^2$ | für Wasserstoff, Fluor, Chlor oder Brom steht, |
| $R^3$ | für Wasserstoff, Fluor, Chlor, Brom oder Trifluormethoxy steht, |
| $R^4$ | für Fluor, Chlor, Brom, Trifluormethoxy, Trifluormethylthio, Trifluormethylsulfinyl, Trifluormethylsulfonyl, Difluormethoxy, Difluorchlormethoxy, Trifluorchlorethoxy, Tetrafluorethoxy, Trifluorethoxy, Difluormethylthio, Trifluorchlorethylthio, Tetrafluorethylthio, Difluormethylsulfinyl, Difluorchlormethylsulfinyl, Trifluorchlorethylsulfinyl, Tetrafluorethylsulfinyl, Difluormethylsulfonyl, Difluorchlormethylsulfonyl, Trifluorchlorethylsulfonyl, Tetrafluorethylsulfonyl, Difluorbrommethylthio, Difluorbrommethylsulfinyl, Difluorbrommethylsulfonyl oder Difluorchlormethylthio steht oder |
| $R^3$ und $R^4$ | gemeinsam für eine Gruppierung der Formel |

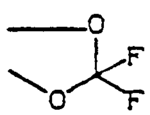 , 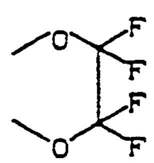 oder 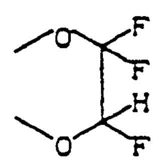

| | |
|---|---|
| | stehen, |
| A | für eine Ketogruppe oder eine CH(OH)-Gruppierung steht, |
| Z | für ein Stickstoffatom oder die CH-Gruppe steht und |
| $R^6$ und $R^7$ | gleich oder verschieden sind und unabhängig voneinander für Wasserstoff, geradkettiges oder verzweigtes Alkyl mit 1 bis 12 Kohlenstoffatomen; geradkettiges oder verzweigtes Alkenyl oder Alkinyl mit jeweils 3 bis 12 Kohlenstoffatomen; gegebenenfalls einfach oder zweifach, gleichartig oder verschieden durch Halogen und/oder Alkyl substituiertes Cycloalkyl mit 3 bis 7 Kohlenstoffatomen; gegebenenfalls einfach oder zweifach, gleichartig oder verschieden durch Halogen und/oder Alkyl substituiertes Cycloalkylalkyl mit 3 bis 7 Kohlenstoffatomen im Cycloalkylteil und 1 bis 4 Kohlenstoffatomen im Alkylteil stehen oder für gegebenenfalls substituiertes Phenylalkyl mit 1 bis 4 Kohlenstoffatomen im Alkylteil stehen, wobei als Phenylsubstituenten Halogen, Alkyl mit 1 bis 4 Kohlenstoffatomen; Alkoxy und Alkylthio mit jeweils 1 bis 4 Kohlenstoffatomen; Halogenalkyl, Halogenalkoxy, Halogenalkylthio, Halogenalkylsulfinyl und Haloge- |

nalkylsulfonyl mit jeweils 1 bis 4 Kohlenstoffatomen und 1 bis 5 gleichen oder verschiedenen Halogenatomen, wie Fluor-, Chlor- und/oder Bromatomen; Nitro, Cyano, Alkoxycarbonyl mit 1 bis 4 Kohlenstoffatomen im Alkoxyteil oder gegebenenfalls einfach bis dreifach, gleich oder verschieden durch Fluor, Chlor und/oder Brom substituiertes Phenyl und Phenoxy genannt seien oder zwei benachbarte Phenylsubstituenten gemeinsam für eine Gruppierung der Formel

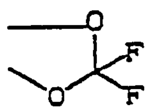 , 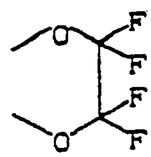 oder 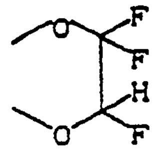

stehen,

wobei jedoch $R^3$ für Trifluormethoxy steht, wenn $R^4$ für Fluor, Chlor oder Brom steht,

sowie deren Säureadditionssalze und Metallsalzkomplexe gefunden.

Die Verbindungen der Formel (I), in denen A für die CH(OH)-Gruppe steht, besitzen zwei benachbarte asymmetrisch substituierte Kohlenstoffatome. Sie können deshalb in den beiden geometrischen Isomeren (threo- und erythroForm) vorliegen, die in unterschiedlichen Mengenverhältnissen anfallen können. Die Verbindungen der Formel (I), in denen A für die Ketogruppe steht, besitzen ein asymmetrisch substituiertes Kohlenstoffatom. In beiden Fällen können die Verbindungen der Formel (I) in verschiedenen optischen Isomerenformen vorliegen, die in unterschiedlichen Mengenverhältnissen anfallen können. Sämtliche Isomeren werden erfindungsgemäß beansprucht.

Weiterhin wurde gefunden, daß man die neuen 1,4-disubstituierten 1-Azolyl-3,3-dimethylbutan-Derivate der Formel (I) sowie deren Säureadditionssalze und Metallsalzkomplexe erhält, wenn man

(a) Halogen-ketone der Formel

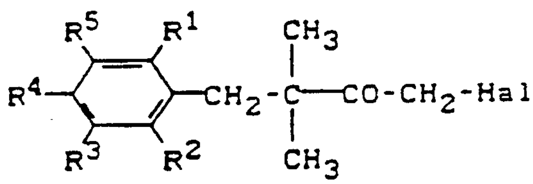 (II)

in welcher

$R^1$, $R^2$, $R^3$, $R^4$, $R^5$ die oben angegebene Bedeutung haben und

Hal für Chlor, Brom oder Iod steht,

mit einem Azol der Formel

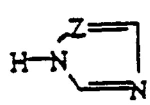 (III)

in welcher

Z die oben angegebene Bedeutung hat,

in Gegenwart eines Verdünnungsmittels und in Gegenwart eines Säurebindemittels umsetzt,

oder

(b) Ketone der Formel

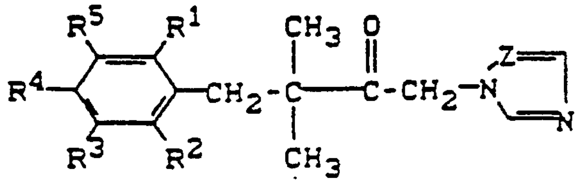

(Ia)

in welcher

$R^1$, $R^2$, $R^3$, $R^4$, $R^5$ und Z die oben angegebene Bedeudeutung haben,

mit einer Verbindung der Formel

$R^8$ - Y (IV)

in welcher

$R^8$ für geradkettiges oder verzweigtes Alkyl mit 1 bis 12 Kohlenstoffatomen; geradkettiges oder verzweigtes Alkenyl oder Alkinyl mit jeweils 3 bis 12 Kohlenstoffatomen; gegebenenfalls einfach oder zweifach, gleichartig oder verschieden durch Halogen und/oder Alkyl substituiertes Cycloalkyl mit 3 bis 7 Kohlenstoffatomen; gegebenenfalls einfach oder zweifach, gleichartig oder verschieden durch Halogen und/oder Alkyl substituiertes Cycloalkylalkyl mit 3 bis 7 Kohlenstoffatomen im Cycloalkylteil und 1 bis 4 Kohlenstoffatomen im Alkylteil steht oder für gegebenenfalls substituiertes Phenylalkyl mit 1 bis 4 Kohlenstoffatomen im Alkylteil steht, wobei als phenylsubstituenten Halogen, Alkyl mit 1 bis 4 Kohlenstoffatomen; Alkoxy und Alkylthio mit jeweils 1 bis 4 Kohlenstoffatomen; Halogenalkyl, Halogenalkoxy, Halogenalkylthio, Halogenalkylsulfinyl und Halogenalkylsulfonyl mit jeweils 1 bis 4 Kohlenstoffatomen und 1 bis 5 gleichen oder verschiedenen Halogenatomen, wie Fluor-, Chlor- und/oder Bromatomen; Nitro, Cyano, Alkoxycarbonyl mit 1 bis 4 Kohlenstoffatomen im Alkoxyteil oder gegebenenfalls einfach bis dreifach, gleich oder verschieden durch Fluor, Chlor und/oder Brom substituiertes Phenyl und Phenoxy genannt seien oder zwei benachbarte Phenylsubstituenten gemeinsam für eine Gruppierung der Formel

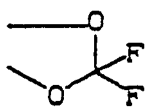

,

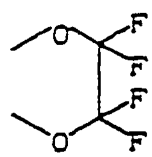

oder

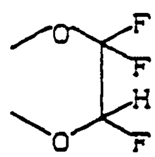

stehen,

und

Y für Chlor, Brom, Iod, p-Methylphenylsulfonyloxy, die Gruppierung $-O-SO_2-OR$ oder $NR_3$ steht, wobei R für Methyl, Ethyl, n- oder i-Propyl steht,

gegebenenfalls in Gegenwart eines Säurebindemittels sowie in Gegenwart eines Verdünnungsmittels und gegebenenfalls in Gegenwart eines Katalysators umsetzt,

oder

(c) Ketone der Formel

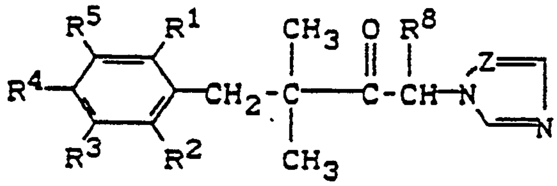

(Ib)

in welcher

$R^1$, $R^2$, $R^3$, $R^4$, $R^5$, $R^8$ und Z die oben angegebene Bedeutung haben,

mit einer Verbindung der Formel

$R^9$ - $Y^1$ (V)

in welcher

$R^9$ für geradkettiges oder verzweigtes Alkyl mit 1 bis 12 Kohlenstoffatomen; geradkettiges oder verzweigtes Alkenyl oder Alkinyl mit jeweils 3 bis 12 Kohlenstoffatomen; gegebenenfalls einfach oder zweifach, gleichartig oder verschieden durch Halogen und/oder Alkyl substituiertes Cycloalkyl mit 3 bis 7 Kohlenstoffatomen; gegebenenfalls einfach oder zweifach, gleichartig oder verschieden durchHalogen und/oder Alkyl substituiertes Cycloalkylalkyl mit 3 bis 7 Kohlenstoffatomen im Cycloalkylteil und 1 bis 4 Kohlenstoffatomen im Alkylteil steht oder für gegebenenfalls substituiertes Phenylalkyl mit 1 bis 4 Kohlenstoffatomen im Alkylteil steht, wobei als Phenylsubstituenten Halogen, Alkyl mit 1 bis 4 Kohlenstoffatomen; Alkoxy und Alkylthio mit jeweils 1 bis 4 Kohlenstoffatomen; Halogenalkyl, Halogenalkoxy, Halogenalkylthio, Halogenalkylsulfinyl und Halogenalkylsulfonyl mit jeweils 1 bis 4 Kohlenstoffatomen und 1 bis 5 gleichen oder verschiedenen Halogenatomen, wie Fluor-, Chlor- und/oder Bromatomen; Nitro, Cyano, Alkoxycarbonyl mit 1 bis 4 Kohlenstoffatomen im Alkoxyteil oder gegebenenfalls einfach bis dreifach, gleich oder verschieden durch Fluor, Chlor und/oder Brom substituiertes Phenyl und Phenoxy genannt seien oder zwei benachbarte Phenylsubstituenten gemeinsam für eine Gruppierung der Formel

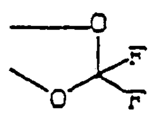 , 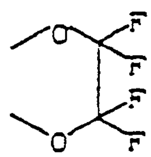 oder 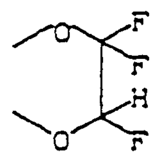

stehen,

und

$Y^1$ für Chlor, Brom, Iod, p-Methylphenylsulfonyloxy, die Gruppierung $-O-SO_2-OR$ oder $NR_3$ steht, wobei R für Methyl, Ethyl, n- oder i-Propyl steht, gegebenenfalls in Gegenwart eines Säurebindemittels sowie in Gegenwart eines Verdünnungsmittels und gegebenenfalls in Gegenwart eines Katalysators umsetzt,

oder

(d) Ketone der Formel

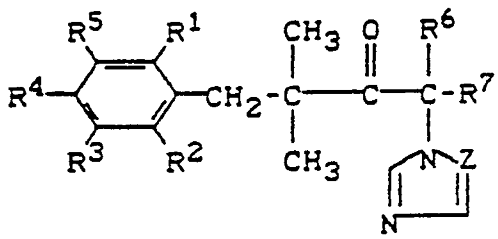 (Ic)

in welcher

$R^1$, $R^2$, $R^3$, $R^4$, $R^5$, $R^6$ und $R^7$ die oben angegebene Bedeutung haben,

mit einem Reduktionsmittel gegebenenfalls in Gegenwart eines Verdünnungsmittels umsetzt,

oder

(e) 1,4-disubstituierte 1-Azolyl-3,3-dimethylbutan-Derivate der Formel

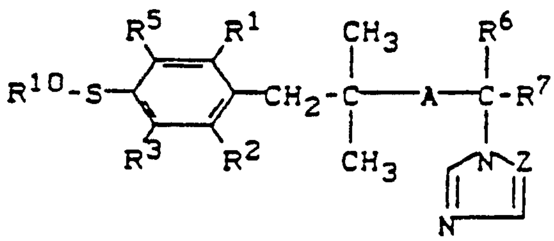

(Id)

in welcher

$R^1$, $R^2$, $R^3$, $R^5$, $R^6$, $R^7$, A und Z die oben angegebene Bedeutung haben und

$R^{10}$ für Trifluormethyl, Difluormethyl, Trifluorchlorethyl, Tetrafluorethyl, Difluorchlormethyl oder Difluorbrommethyl steht,

mit einem Oxidationsmittel, gegebenenfalls in Gegenwart eines Verdünnungsmittels und gegebenenfalls in Gegenwart eines Reaktionshilfsmittels umsetzt,

und gegebenenfalls an die so erhaltenen Verbindungen der Formel (I) eine Säure oder ein Metallsalz addiert.

Schließlich wurde gefunden, daß die neuen 1,4-disubstituierten 1-Azolyl-3,3-dimethylbutan-Derivate der Formel (I) sowie deren Säureadditions-Salze und MetallsalzKomplexe sehr gute fungizide Eigenschaften besitzen.

Überraschenderweise zeichnen sich die erfindungsgemäßen Stoffe durch eine bessere fungizide Wirkung aus als die konstitutionell ähnlichen, aus dem Stand der Technik bekannten Verbindungen gleicher Indikation. So übertreffen die erfindungsgemäßen Stoffe zum Beispiel 1-(4-Chlorphenyl)-2,2-dimethyl-4-(1,2,4-triazol-1-yl)-hexan-3-on; 1-(4-Chlorphenyl)-2,2-dimethyl-4-(1,2,4-triazol-1-yl)-hexan-3-ol; 1-(3-Brom-4-fluorphenyl)-2,2-dimethyl-4-(1,2,4-triazol-1-yl)-oct-7-en-3-ol und 1-(4-Methylphenyl )-2,2-dimethyl-4-(1,2,4-triazol-1-yl)-pentan-3-ol bezüglich ihrer fungiziden Eigenschaften.

Außerdem sind die neuen 1,4-disubstituierten 1-Azolyl-3,3-dimethyl-butan-2-one der Formeln (Ia), (Ib), (Ic) und (Id) interessante Zwischenprodukte zur Herstellung von weiteren Pflanzenschutz-Wirkstoffen. So Können durch entsprechende Umsetzungen funktionelle Derivate der Ketogruppe erhalten werden wie z. B. Oxime und Oximether, Hydrazone und Ketale.

Im übrigen sind auch die neuen 1,4-disubstituierten 1-Azolyl-3,3-dimethyl-butan-2-ole interessante Zwischenprodukte zur Herstellung von weiteren PflanzenschutzWirkstoffen. So können z. B. diese Verbindungen durch Umsetzung an der Hydroxy-Gruppe in üblicher Weise in die entsprechenden Ether überführt werden. Weiterhin Können durch Umsetzung mit z. B. Acylhalogeniden oder Carbamoylchloriden in prinzipiell bekannter Weise Acyl- oder Carbamoyl-Derivate der Alkohole der Formel (I) erhalten werden.

Die erfindungsgemäßen 1,4-disubstituierten 1-Azolyl-3,3-dimethylbutan-Derivate sind durch die Formel (I) allgemein definiert.

Bevorzugt sind Verbinungen der Formel (I), in denen

$R^1$ für Wasserstoff, Fluor, Chlor oder Brom steht,

$R^2$ für Wasserstoff, Fluor, Chlor oder Brom steht,

$R^3$ für Wasserstoff, Fluor, Chlor, Brom oder Trifluormethoxy steht,

$R^4$ für Fluor, Chlor, Brom, Trifluormethoxy, Trifluormethylthio, Trifluormethylsulfinyl, Trifluormethylsulfonyl, Difluormethoxy, Difluorchlormethoxy, Trifluorchlorethoxy, Tetrafluorethoxy, Trifluorethoxy, Difluormethylthio, Trifluorchlorethylthio, Tetrafluorethylthio, Difluormethylsulfinyl, Difluorchlormethylsulfinyl, Trifluorchlorethylsulfinyl, Tetrafluorethylsulfinyl, Difluormethylsulfonyl, Difluorchlormethylsulfonyl, Trifluorchlorethylsulfonyl, Tetrafluorethylsulfonyl, Difluorbrommethylthio, Difluorbrommethylsulfinyl, Difluorbrommethylsulfonyl oder Difluorchlormethylthio steht oder

$R^3$ und $R^4$ gemeinsam für eine Gruppierung der Formel

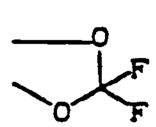
,
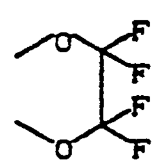
oder
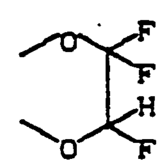

stehen,

A für eine Ketogruppe oder eine CH(OH)-Gruppierung steht,

Z für ein Stickstoffatom oder die CH-Gruppe steht,

$R^6$ für Wasserstoff, geradkettiges oder verzweigtes Alkyl mit 1 bis 7 Kohlenstoffatomen; geradkettiges oder verzweigtes Alkenyl oder Alkinyl mit jeweils 3 bis 7 Kohlenstoffatomen; gegebenenfalls ein- oder zweifach, gleichartig oder verschieden durch Fluor, Chlor und/oder Methyl substituiertes Cycloalkyl mit 5 bis 7 Kohlenstoffatomen; gegebenenfalls ein- oder zweifach, gleichartig oder verschieden durch Fluor, Chlor und/oder Methyl substituiertes Cycloalkylalkyl mit 3 bis 7 Kohlenstoffatomen im CycloalKylteil und 1 bis 4 Kohlenstoffatomen im Alkylteil; oder für Phenylalkyl mit 1 oder 2 Kohlenstoffatomen im Alkylteil steht, wobei der Phenylteil einfach bis dreifach substituiert sein kann durch Fluor, Chlor, Brom, Methyl, Ethyl, n-Propyl, tert.-Butyl, Methoxy, Ethoxy, Methylthio, Ethylthio, Trifluormethyl, Trifluormethoxy, Trifluormethylthio, Trifluormethylsulfinyl, Trifluormethylsulfonyl, Nitro, Cyano, Methoxycarbonyl, Ethoxycarbonyl, gegebenenfalls durch Fluor und/oder Chlor substituiertes Phenyl und/oder durch Fluor und/oder Chlor substituiertes Phenoxy, oder durch zwei benachbarte Substituenten, die gemeinsam für eine Gruppierung der Formel

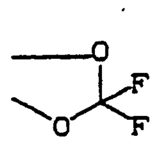 , 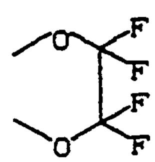 oder 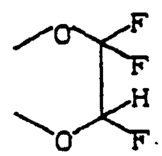

stehen,

und

$R^7$ für Wasserstoff, geradkettiges oder verzweigtes Alkyl mit 1 bis 7 Kohlenstoffatomen; geradkettiges oder verzweigtes Alkenyl oder Alkinyl mit jeweils 3 bis 7 Kohlenstoffatomen; gegebenenfalls ein- oder zweifach, gleichartig oder verschieden durch Fluor, Chlor und/oder Methyl substituiertes Cycloalkyl mit 5 bis 7 Kohlenstoffatomen; gegebenenfalls ein- oder zweifach, gleichartig oder verschieden durch Fluor, Chlor und/oder Methyl substituiertes Cycloalkylalkyl mit 3 bis 7 Kohlenstoffatomen im Cycloalkylteil und 1 bis 4 Kohlenstoffatomen im Alkylteil; oder für Benzyl steht, wobei der Phenylteil einfach bis dreifach, gleichartig oder verschieden substituiert sein kann durch Fluor, Chlor, Brom, Methyl, Ethyl, n-Propyl, tert.-Butyl, Methoxy, Ethoxy, Methylthio, Ethylthio, Trifluormethyl, Trifluormethoxy, Trifluormethylthio, Trifluormethylsulfinyl, Trifluormethylsulfonyl, Nitro, Cyano, Methoxycarbonyl, Ethoxycarbonyl, gegebenenfalls durch Fluor und/oder Chlor substituiertes Phenyl und/oder durch Fluor und/oder Chlor substituiertes Phenoxy, oder durch zwei benachbarte Substituenten, die gemeinsam für eine Gruppierung der Formel

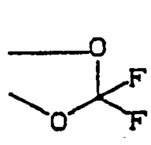 , 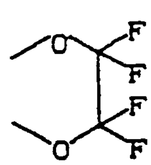 oder 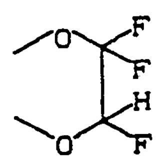

stehen,

wobei jedoch $R^3$ für Trifluormethoxy steht, wenn $R^4$ für Fluor, Chlor oder Brom steht.

Bevorzugte erfindungsgemäße Verbindungen sind auch Additionsprodukte aus Säuren und denjenigen 1,4-disubstituierten 1-Azolyl-3,3-dimethyl-butan-Derivaten der Formel (I), in denen die Substituenten $R^1$, $R^2$, $R^3$, $R^4$, $R^5$, $R^6$, $R^7$, A und Z die Bedeutungen haben, die bereits vorzugsweise für diese Substituenten genannt wurden.

Zu den Säuren, die addiert werden Können, gehören vorzugsweise Halogenwasserstoffsäuren wie z. B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren wie z. B. Essigsäure, Maleinsäure, Bernsteinsäure, Fumarsäure, Weinsäure, Zitronensäure, Salizylsäure, Sorbinsäure

und Milchsäure, Sulfonsäuren wie z. B. pToluolsulfonsäure und 1,5-Naphthalindisulfonsäure sowie Saccharin und Thiosaccharin.

Außerdem bevorzugte erfindungsgemäße Verbindungen sind Additionsprodukte aus Salzen von Metallen der II. bis IV. Haupt- und der I und II. sowie IV. bis VIII. Nebengruppe des Periodensystems der Elemente und denjenigen Verbindungen der Formel (I), in denen die Substituenten $R^1$, $R^2$, $R^3$, $R^4$, $R^5$, $R^6$, $R^7$, A und Z die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugt derartige Säuren sind in diesem Zusammenhang die Halogenwasserstoffsäuren wie z. B. die Chlorwasserstoffsäure und die Bromwasserstoffsäure, ferner Phosphorsäure, Salpetersäure und Schwefelsäure.

Im einzelnen seien außer den bei den Herstellungsbeispielen genannten Verbindungen die folgenden Verbindungen der Formel

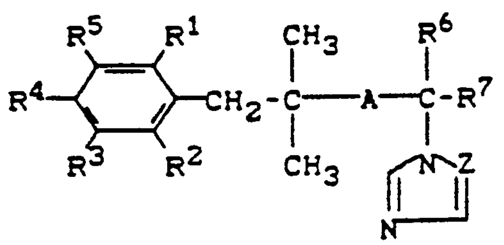

(I)

in welcher

A für eine Ketogruppe oder eine CH(OH)-Gruppierung steht und

Z für ein Stickstoffatom oder die CH-Gruppe steht,

genannt:

<u>**Tabelle 1**</u>

| $R^1$ | $R^2$ | $R^3$ | $R^4$ | $R^5$ | $R^6$ | $R^7$ |
|---|---|---|---|---|---|---|
| H | H | Cl | $-OCF_3$ | H | H | $-CH_2-C \equiv CH$ |
| H | H | Cl | $-OCF_3$ | H | H | $-CH_2$ 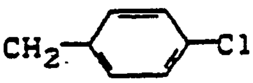 |
| H | H | Cl | $-OCF_3$ | H | H | $-CH_2$ 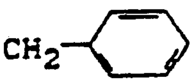 |
| H | H | Cl | $-OCF_2Cl$ | H | H | $-CH_3$ |
| H | H | Cl | $-OCF_2Cl$ | H | H | $-C_2H_5$ |
| H | H | Cl | $-OCF_2Cl$ | H | H | $-C_3H_7-n$ |
| H | H | Cl | $-OCF_2Cl$ | H | H | $-C_4H_9$ |
| H | H | Cl | $-OCF_2Cl$ | H | H | $-CH_2-CH=CH_2$ |
| H | H | Cl | $-OCF_2Cl$ | H | H | $-CH_2-C \equiv CH$ |
| H | H | Cl | $-OCF_2Cl$ | H | H | $-CH_2$ 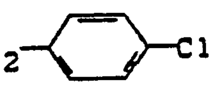 |
| H | H | Cl | $-OCF_2Cl$ | H | H | $-CH_2$ 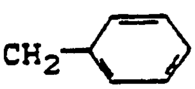 |
| H | H | F | $-OCF_3$ | H | H | $-CH_3$ |
| H | Cl | H | $-OCF_3$ | H | H | $-CH_2-CH=CH_2$ |
| H | F | H | $-OCF_3$ | H | H | $-CH_3$ |
| H | F | H | $-OCF_3$ | H | H | $-CH_2-CH=CH_2$ |

Tabelle 1 (Fortsetzung)

| $R^1$ | $R^2$ | $R^3$ | $R^4$ | $R^5$ | $R^6$ | $R^7$ |
|---|---|---|---|---|---|---|
| H | H | H | $-OCHF_2$ | H | H | $-C_2H_5$ |
| H | H | H | $-OCHF_2$ | H | H | $-C_3H_7$-n |
| H | H | H | $-OCF_2CHF_2$ | H | H | $-CH_3$ |
| H | Cl | H | $-OCF_2CHF_2$ | H | H | $-CH_3$ |
| H | F | H | $-OCF_2CHF_2$ | H | H | $-CH_3$ |
| H | H | Cl | $-OCF_2CHF_2$ | H | H | $-CH_3$ |
| H | H | F | $-OCF_2CHF_2$ | H | H | $-CH_3$ |
| H | H | H | $-OCF_2CHF_2$ | H | H | $-C_2H_5$ |
| H | H | H | $-OCF_2CHF_2$ | H | H | $-C_3H_7$-n |
| H | H | H | $-OCF_2CHF_2$ | H | H | $-C_4H_9$-n |
| H | H | H | $-OCF_2CHF_2$ | H | H | $-CH_2-CH=CH_2$ |
| H | H | H | $-OCF_2CHF_2$ | H | H | $-CH_2-C\equiv CH$ |
| H | H | H | $-OCF_2CHF_2$ | H | H | $-CH_2-CH=CH-CH_3$ |
| H | H | H | $-OCF_2CHF_2$ | H | H | $-CH_2-C(CH_3)=CH_2$ |
| H | H | H | $-OCF_2CHFCl$ | H | H | $-CH_3$ |
| H | H | H | $-OCF_2CHFCl$ | H | H | $-CH_2-CH=CH-CH_3$ |

Tabelle 1 (Fortsetzung)

| $R^1$ | $R^2$ | $R^3$ | $R^4$ | $R^5$ | $R^6$ | $R^7$ |
|---|---|---|---|---|---|---|
| H | H | Cl | $-OCF_2CHFCl$ | H | H | $-CH_3$ |
| H | H | H | $-OCH_2CF_3$ | H | H | $-CH_3$ |
| H | H | Cl | $-SCHF_2$ | H | H | $-CH_3$ |
| H | H | H | $-SCHF_2$ | H | H | $-CH_3$ |
| H | H | H | $-SCF_2CHFCl$ | H | H | $-CH_3$ |
| H | H | H | $-SCF_2CHF_2$ | H | H | $-CH_3$ |
| H | H | H | $-SCF_3$ | H | H | $-C_2H_5$ |
| H | H | H | $-SCF_3$ | H | H | $-C_3H_7-n$ |
| H | H | H | $-SCF_3$ | H | H | $-C_4H_9-n$ |
| H | H | H | $-SCF_3$ | H | H | $-CH_2-CH=CH_2$ |
| H | H | H | $-SCF_3$ | H | H | $-CH_2-CH=CH-CH_3$ |
| H | H | H | $-SCF_3$ | H | H | $-CH_2-C(CH_3)=CH_2$ |
| H | H | H | $-SCF_3$ | H | H | $-CH_2$ 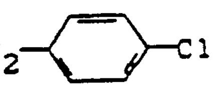 |
| H | H | H | $-SCF_3$ | H | H | $-CH_2$ 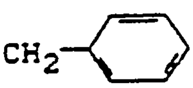 |
| H | H | Cl | $-SCF_3$ | H | H | $-CH_3$ |
| H | H | Cl | $-SOCHF_2$ | H | H | $-CH_3$ |

<u>Tabelle 1</u> (Fortsetzung)

| $R^1$ | $R^2$ | $R^3$ | $R^4$ | $R^5$ | $R^6$ | $R^7$ |
|---|---|---|---|---|---|---|
| H | H | H | $-SOCHF_2$ | H | H | $-CH_3$ |
| H | H | H | $-SOCF_2CHFCl$ | H | H | $-CH_3$ |
| H | H | H | $-SOCF_2CHF_2$ | H | H | $-CH_3$ |
| H | H | H | $-SOCF_3$ | H | H | $-C_2H_5$ |
| H | H | H | $-SOCF_3$ | H | H | $-C_3H_7-n$ |
| H | H | H | $-SOCF_3$ | H | H | $-C_4H_9-n$ |
| H | H | H | $-SOCF_3$ | H | H | $-CH_2-CH=CH_2$ |
| H | H | H | $-SOCF_3$ | H | H | $-CH_2-CH=CH-CH_3$ |
| H | H | H | $-SOCF_3$ | H | H | $-CH_2-\underset{CH_3}{\underset{\vert}{C}}=CH_2$ |
| H | H | H | $-SOCF_3$ | H | H | $-CH_2$ 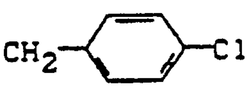 |
| H | H | H | $-SOCF_3$ | H | H | $-CH_2$ 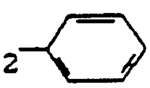 |
| H | H | Cl | $-SOCF_3$ | H | H | $-CH_3$ |
| H | H | Cl | $-SO_2CHF_2$ | H | H | $-CH_3$ |
| H | H | H | $-SO_2CHF_2$ | H | H | $-CH_3$ |
| H | H | H | $-SO_2CF_2CHFCl$ | H | H | $-CH_3$ |
| H | H | H | $-SO_2CF_2CHF_2$ | H | H | $-CH_3$ |

Tabelle 1 (Fortsetzung)

| $R^1$ | $R^2$ | $R^3$ | $R^4$ | $R^5$ | $R^6$ | $R^7$ |
|---|---|---|---|---|---|---|
| H | H | H | $-SO_2CF_3$ | H | H | $-C_2H_5$ |
| H | H | H | $-SO_2CF_3$ | H | H | $-C_3H_7$-n |
| H | H | H | $-SO_2CF_3$ | H | H | $-C_4H_9$-n |
| H | H | H | $-SO_2CF_3$ | H | H | $-CH_2-CH=CH_2$ |
| H | H | H | $-SO_2CF_3$ | H | H | $-CH_2-CH=CH-CH_3$ |
| H | H | H | $-SO_2CF_3$ | H | H | 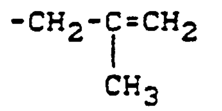 |
| H | H | H | $-SO_2CF_3$ | H | H | 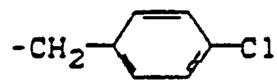 |
| H | H | H | $-SO_2CF_3$ | H | H | 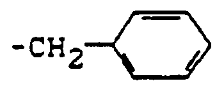 |
| H | H | Cl | $-SO_2CF_3$ | H | H | $-CH_3$ |
| H | H | H | 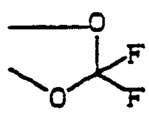 | | H | $-CH_3$ |
| H | H | H | 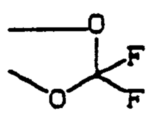 | | H | $-C_2H_5$ |
| H | H | H | 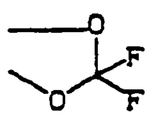 | | H | $-C_3H_7$-n |
| H | H | H | 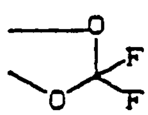 | | H | $-CH_2-CH=CH_2$ |

Tabelle 1 (Fortsetzung)

| $R^1$ | $R^2$ | $R^3$ | $R^4$ | $R^5$ | $R^6$ | $R^7$ |
|---|---|---|---|---|---|---|
| H | H | H | 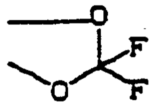 | | H | $-CH_2-CH=CH-CH_3$ |
| H | H | Br | $-OCF_3$ | H | H | $-CH_3$ |
| H | H | Br | $-OCF_3$ | H | H | $-C_2H_5$ |
| H | H | Br | $-OCF_3$ | H | H | $-C_3H_7-n$ |
| H | H | Br | $-OCF_3$ | H | H | $-C_4H_9-n$ |
| H | H | Br | $-OCF_3$ | H | H | $-CH_2-CH=CH_2$ |
| H | H | Br | $-OCF_3$ | H | H | $-CH_2-C\equiv CH$ |
| H | H | Br | $-OCF_3$ | H | H | $-CH_2-C(CH_3)=CH_2$ |
| H | H | Br | $-OCF_3$ | H | H | $-CH_2-CH=CH-CH_3$ |
| H | H | H | 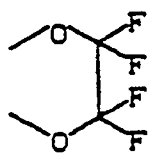 | | H | $-CH_3$ |
| H | H | H | 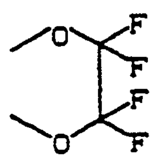 | | H | $-C_2H_5$ |

Tabelle 1 (Fortsetzung)

| $R^1$ | $R^2$ | $R^3$ | $R^4$ | $R^5$ | $R^6$ | $R^7$ |
|---|---|---|---|---|---|---|
| H | H | H | 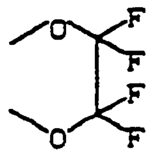 | | H | $-C_3H_7-n$ |
| H | H | H | 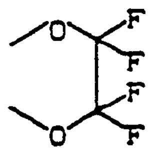 | | H | $-C_4H_9-n$ |
| H | H | H | 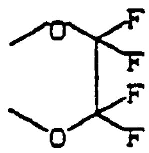 | | H | $-CH_2-CH=CH_2$ |
| H | H | H | 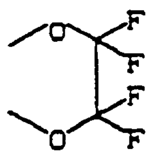 | | H | $-CH_2-CH=CH-CH_3$ |
| H | H | H | 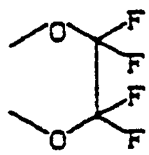 | | H | $-CH_2-C=CH_2$<br>$\quad\ \ |$<br>$\quad CH_3$ |
| H | H | H | $-SCF_3$ | H | $CH_3$ | $-CH_3$ |
| H | H | H | $-SOCF_3$ | H | $CH_3$ | $-CH_3$ |
| H | H | H | $-SO_2CF_3$ | H | $CH_3$ | $-CH_3$ |
| H | H | Cl | $-OCF_3$ | H | $CH_3$ | $-CH_3$ |
| H | H | Br | $-OCF_3$ | H | $CH_3$ | $-CH_3$ |

Tabelle 1 (Fortsetzung)

| $R^1$ | $R^2$ | $R^3$ | $R^4$ | $R^5$ | $R^6$ | $R^7$ |
|---|---|---|---|---|---|---|
| H | H | H | $-OCF_2Cl$ | H | $CH_3$ | $-CH_3$ |
| H | H | H | $-SCF_2Cl$ | H | $CH_3$ | $-CH_3$ |
| H | H | H | $-SOCF_2Cl$ | H | $CH_3$ | $-CH_3$ |
| H | H | H | $-SO_2CF_2Cl$ | H | $CH_3$ | $-CH_3$ |
| H | H | H | $-SCF_2Br$ | H | H | $-CH_3$ |
| H | H | H | $-SOCF_2Br$ | H | H | $-CH_3$ |
| H | H | H | $-SO_2CF_2Br$ | H | H | $-CH_3$ |
| H | H | H | $-SCF_2Br$ | H | $CH_3$ | $-CH_3$ |
| H | H | H | $-SOCF_2Br$ | H | $CH_3$ | $-CH_3$ |
| H | H | H | $-SO_2CF_2Br$ | H | $CH_3$ | $-CH_3$ |
| H | H | H | 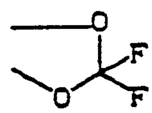 | | $CH_3$ | $-CH_3$ |
| H | H | H | 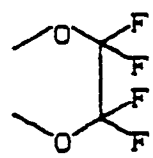 | | $CH_3$ | $-CH_3$ |
| H | H | H | 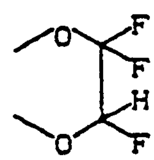 | | $CH_3$ | $-CH_3$ |
| H | H | Cl | $-OCF_2Cl$ | H | $CH_3$ | $-CH_3$ |
| H | H | H | $-OCHF_2$ | H | $CH_3$ | $-CH_3$ |
| H | H | H | $-OCF_2CHF_2$ | H | $CH_3$ | $-CH_3$ |
| H | H | H | $-OCF_2CHFCl$ | H | $CH_3$ | $-CH_3$ |

Tabelle 1 (Fortsetzung)

| $R^1$ | $R^2$ | $R^3$ | $R^4$ | $R^5$ | $R^6$ | $R^7$ |
|---|---|---|---|---|---|---|
| H | H | H | $-SCHF_2$ | H | $CH_3$ | $-CH_3$ |
| H | H | H | $-SOCHF_2$ | H | $CH_3$ | $-CH_3$ |
| H | H | H | $-SO_2CHF_2$ | H | $CH_3$ | $-CH_3$ |
| H | H | H | $-SCF_2CHF_2$ | H | $CH_3$ | $-CH_3$ |
| H | H | H | $-SOCF_2CHF_2$ | H | $CH_3$ | $-CH_3$ |
| H | H | H | $-SO_2CF_2CHF_2$ | H | $CH_3$ | $-CH_3$ |
| H | H | H | $-SCF_2CHFCl$ | H | $CH_3$ | $-CH_3$ |
| H | H | H | $-SOCF_2CHFCl$ | H | $CH_3$ | $-CH_3$ |
| H | H | H | $-SO_2CF_2CHFCl$ | H | $CH_3$ | $-CH_3$ |
| H | F | H | 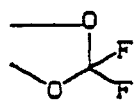 | | H | $-CH_3$ |
| H | Cl | H | 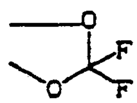 | | H | $-C_2H_5$ |
| H | F | H | 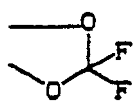 | | $CH_3$ | $-CH_3$ |
| H | Cl | H | 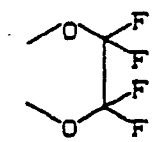 | | $CH_3$ | $-CH_3$ |
| H | Cl | H | 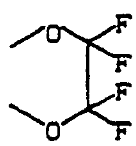 | | $CH_3$ | $-CH_3$ |
| H | F | H | 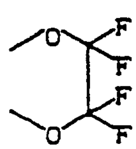 | | H | $-CH_3$ |
| H | Cl | H | 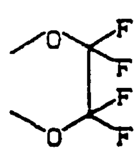 | | H | $-C_2H_5$ |

Verwendet man beispielsweise 1-Brom-4-(4-chlor-3-trifluormethylphenyl)-3,3-dimethyl-butan-2-on und 1,2,4-Triazol als Ausgangsstoffe, so kann der Ablauf des erfindungsgemäßen Verfahrens (a) durch das folgende Formelschema wiedergegeben werden:

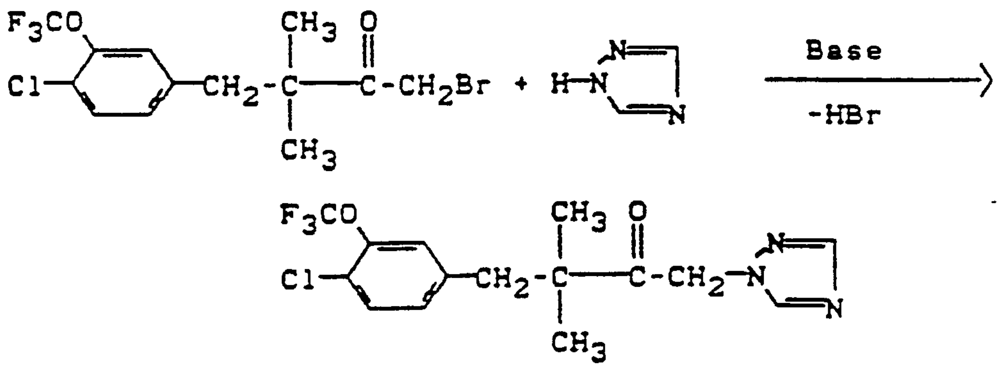


Verwendet man beispielsweise 4-(2,6-Dichlor-4-trifluormethoxyphenyl)-3,3-dimethyl-1-1(1,2,4-triazol-1-yl)-butan-2-on und 4-Chlor-benzylchlorid als Ausgangsstoffe, so kann der Ablauf des erfindungsgemäßen Verfahrens (b) durch das folgende Formelschema wiedergegeben werden:

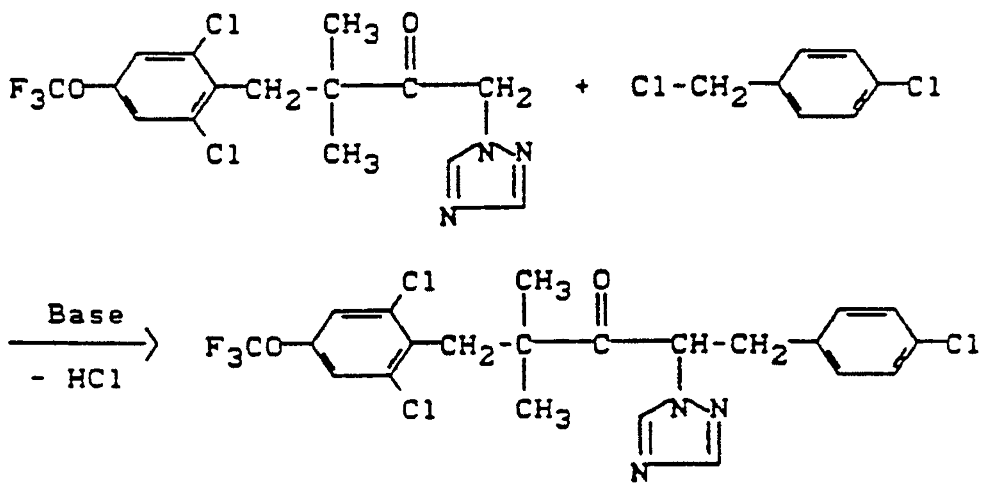


Verwendet man beispielsweise 5-(2,6-Dichlor-4-trifluormethoxy-phenyl)-1-(4-chlorphenyl)-4,4-dimethyl-2-(1,2,4-triazol-1-yl)-pentan-3-on und Methyliodid als Ausgangsstoffe, so kann der Ablauf des erfindungsgemäßen Verfahrens (c) durch das folgende Formelschema wiedergegegen werden:

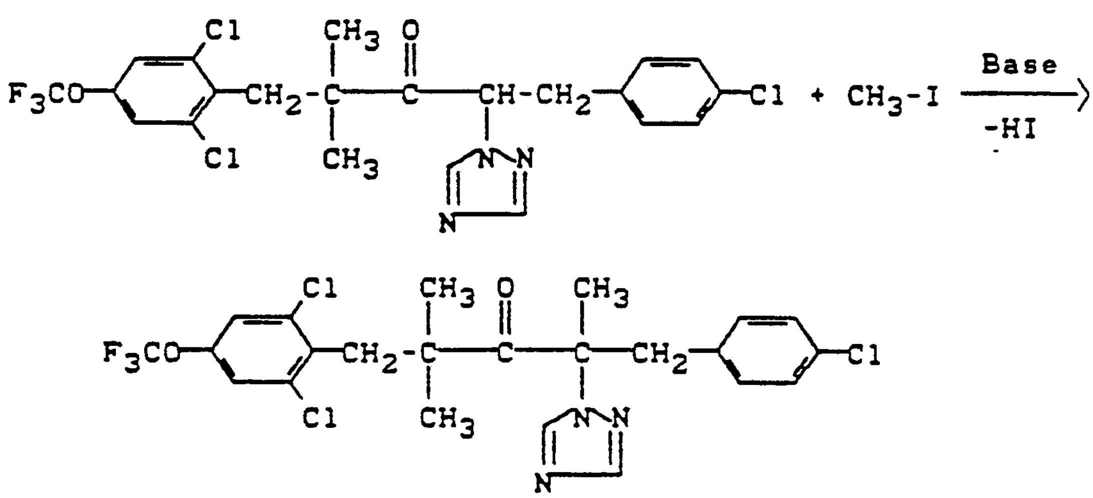


Verwendet man beispielsweise 5-(2,6-Dichlor-4-trifluormethoxy-phenyl)-1-(4-chlorphenyl)-2,4,4-trimethyl-2-(1,2,4-triazol-1-yl)-pentan-3-on als Ausgangsstoff und Natriumborhydrid als Reduktionsmittel, so kann der Ablauf des erfindungsgemäßen Verfahrens (d) durch das folgende Formelschema wiedergegeben werden:

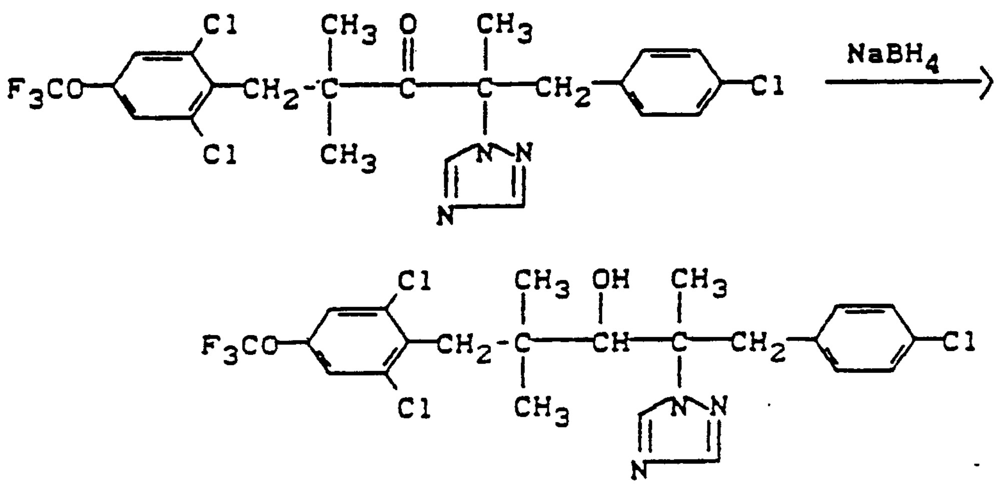


Verwendet man beispielsweise 5-(1-Chlor-3-trifluormethylthiophenyl)-4,4-dimethyl-2-(1,2,4-triazol-1-yl)-pentan-3-on als Ausgangsstoff und Wasserstoffperoxid als Oxidationsmittel, so kann der Ablauf des erfindungsgemäßen Verfahrens (e) durch das folgende Formelschema wiedergegeben werden:

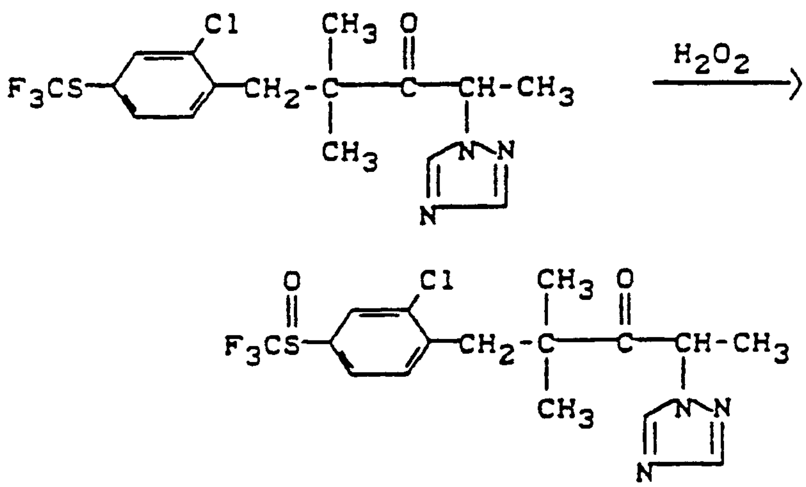


Die bei der Durchführung des erfindungsgemäßen Verfahrens (a) als Ausgangsstoffe benötigten Halogen-ketone sind durch die Formel (II) allgemein definiert. In dieser Formel haben $R^1$, $R^2$, $R^3$, $R^4$ und $R^5$ vorzugsweise diejenigen Bedeutungen, die bereits im Zusammenhang mit der Beschreibung der erfindungsgemäßen Stoffe der Formel (I) vorzugsweise für diese Reste genannt wurden.

Die Halogenketone der Formel (II) sind teilweise bekannt (vgl. DE-OS 3 048 266). Sie lassen sich herstellen, indem man Ketone der Formel

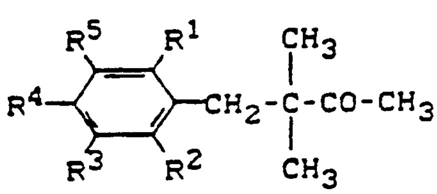
(VI)

in welcher

$R^1$, $R^2$, $R^3$, $R^4$ und $R^5$ die oben angegebene Bedeutung haben,
mit Chlor oder Brom in Gegenwart eines inerten organischen Lösungsmittels, wie beispielsweise Ether, chlorierten oder nicht chlorierten Kohlenwasserstoffen, bei Raumtemperatur umsetzt, oder mit üblichen Chlorierungsmitteln, wie beispielsweise Sulfurylchlorid, bei Temperaturen zwischen 20 °C und 60 °C umsetzt. Die Chlorund Brom-ketone lassen sich nach üblichen Verfahren in die entsprechenden Iod-Verbindungen überführen.

Die Ketone der Formel (VI) sind teilweise bekannt (vgl. DE-OS 30 48 266 und DE-OS 32 10 725). Sie können erhalten werden, indem man Methylisopropylketon mit Halogen-Verbindungen der Formel

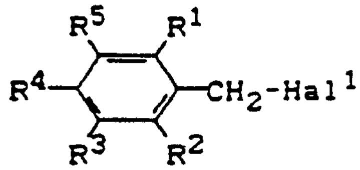

(VII)

in welcher
$R^1$, $R^2$, $R^3$, $R^4$, und $R^5$ die oben angegebene Bedeutung haben und
$Hal^1$ für Halogen, wie Chlor oder Brom, steht,
in Gegenwart einer Base, wie beispielsweise einem Alkali- oder Erdalkalimetallhydroxid, wie pulverisiertes Kalium- und Natriumhydroxid, und in Gegenwart eines Verdünnungsmittels, wie beispielsweise eines (cyclo)aliphatischen, gegebenenfalls chlorierten aromatischen Kohlenwasserstoffes, wie Benzol, Xylol, Chlorbenzol, Dichlorbenzol, Cyclohexan, Petrolether, Pentan, Hexan oder Toluol, sowie in Gegenwart eines Phasentransferkatalysators, wie beispielsweise eines Derivates von Ammoniumsalzen, wie Triethylbenzylammonium-chlorid, Tetrabutylammonium-iodid, -bromid oder -chlorid, bei Temperaturen zwischen 0 °C und 150 °C umsetzt.

Methylisopropylketon und die Halogen-Verbindungen der Formel (VII) sind allgemein bekannte Verbindungen der organischen Chemie.

Die außerdem für das erfindungsgemäße Verfahren (a) als Ausgangsstoffe zu verwendenden Azole der Formel (III) sind ebenfalls allgemein bekannte Verbindungen der organischen Chemie.

Zur Durchführung des erfindungsgemäßen Verfahrens (a) kommen als Verdünnungsmittel inerte organische Lösungsmittel in Frage. Hierzu gehören vorzugsweise Ketone, wie Diethylketon, Aceton und Methylethylketon; Nitrile, wie Propionitril und Acetonitril; Alkohole, wie Ethanol oder Isopropanol; Ether, wie Tetrahydrofuran oder Dioxan; aromatische Kohlenwasserstoffe, wie Toluol, Benzol oder Chlorbenzol; Formamide, wie Dimethylformamid; und halogenierte Kohlenwasserstoffe.

Als Säurebindemittel kommen bei der Durchführung des erfindungsgemäßen Verfahrens (a) alle üblichen anorganischen und organischen Säureakzeptoren in Frage. Vorzugsweise verwendbar sind Alkalimetallcarbonate, wie Natriumcarbonat, Kaliumcarbonat und Natriumhydrogencarbonat, ferner niedere tertiäre Alkylamine, Cycloalkylamine oder Aralkylamine, wie Triethylamin, N,N-Dimethylcyclohexylamin, Dicyclohexylamin, N,N-Dimethylbenzylamin, weiterhin Pyridin und Diazabicyclooctan. Vorzugsweise verwendbar ist auch ein Überschuß an Azol.

Die Reaktionstemperaturen können bei der Durchführung des erfindungsgemäßen Verfahrens (a) in einem größeren Bereich variiert werden. Im allgemeinen arbeitet man zwischen 20 °C und 150 °C, vorzugsweise zwischen 40 °C und 120 °C.

Bei der Durchführung des erfindungsgemäßen Verfahrens (a) setzt man auf 1 Mol Halogenketon der Formel (II) vorzugsweise 2 bis 4 Mol Azol der Formel (III) und 1 bis 4 Mol Säurebinder ein. Zur Isolierung der Verbindungen der Formel (I) verfährt man nach üblichen Methoden. Im allgemeinen geht man so vor, daß man das Lösungsmittel abdestilliert und den Rückstand in üblicher Weise aufarbeitet.

Die als Ausgangsstoffe für die Durchführung des erfindungsgemäßen Verfahrens (b) benötigten Verbindungen der Formel (Ia) sind erfindungsgemäße Verbindungen, die nach dem Verfahren (a) erhältlich sind.

Die außerdem für das erfindungsgemäße Verfahren (b) als Ausgangsstoffe zu verwendenden Verbindungen sind durch die Formel (IV) allgemein definiert. In dieser Formel steht $R^8$ vorzugsweise für diejenigen Reste, die bereits im Zusammenhang mit der Beschreibung der erfindungsgemäßen Stoffe der Formel (I) vorzugsweise für $R^6$ genannt wurden, wobei Wasserstoff ausgenommen ist.

Die Verbindungen der Formel (IV) sind allgemein bekannte Stoffe der organischen Chemie.

Für das erfindungsgemäße Verfahren (b) kommen als Verdünnungsmittel inerte organische Lösungsmittel in Frage. Vorzugsweise verwendbar sind aromatische Kohlenwasserstoffe, wie Benzol, Toluol und Xylol;

halogenierte Kohlenwasserstoffe, wie Methylenchlorid, Tetrachlorkohlenstoff, Chloroform oder Chlorbenzol; Ester, wie Essigester; Formamide, wie Dimethylformamid, sowie Dimethylsulfoxid.

Das erfindungsgemäße Verfahren (b) wird im allgemeinen in Gegenwart eines Säurebindemittels durchgeführt. Hierbei können alle üblichen organischen und insbesondere anorganischen Basen eingesetzt werden. Vorzugsweise verwendbar sind Alkalimetallhydroxide oder Alkalimetallcarbonate, wie Natrium-und Kaliumhydroxid, Natriumcarbonat und Kaliumcarbonat.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 120 °C, vorzugsweise zwischen 20 °C und 100 °C.

Bei der Durchführung des erfindungsgemäßen Verfahrens (b) setzt man vorzugsweise auf 1 Mol einer Verbindung der Formel (Ia) 1 bis 1,5 Mol bzw. 2 bis 3,0 Mol einer Verbindung der Formel (IV) ein. Die Isolierung der Endprodukte der Formel (I) erfolgt nach üblichen Methoden.

Das erfindungsgemäße Verfahren (b) kann auch in einem Zweiphasensystem, wie beispielsweise einem System aus wäßriger Natron- oder Kalilauge/Toluol oder Methylenchlorid, gegebenenfalls unter Zusatz von 0,1 bis 1 Mol eines Phasen-Transfer-Katalysators, beispielsweise einer Ammonium- oder Phosphoniumverbindung, wie Benzyldodecyldimethyl-ammoniumchlorid oder Triethyl-benzyl-ammoniumchlorid, durchgeführt werden.

Die bei dem Verfahren (c) als Ausgangsstoffe benötigten Azolylketone der Formel (Ib) sind erfindungsgemäße Verbindungen, die nach dem Verfahren (b) erhältlich sind. In dieser Formel haben $R^1$, $R^2$, $R^3$, $R^4$, $R^5$ und Z vorzugsweise diejenigen Bedeutungen, die bereits im Zusammenhang mit der Beschreibung der erfindungsgemäßen Stoffe der Formel (I) als bevorzugt genannt sind, und $R^8$ hat vorzugsweise die Bedeutung, die bei der Beschreibung der erfindungsgemäßen Stoffe der Formel (I) vorzugsweise für $R^6$ angegeben wurde, wobei Wasserstoff ausgenommen ist.

Die außerdem für das erfindungsgemäße Verfahren (c) als Ausgangsstoffe zu verwendenden Verbindungen sind durch die Formel (V) allgemein definiert. In dieser Formel steht $R^9$ vorzugsweise für diejenigen Reste, die bereits im Zusammenhang mit der Beschreibung der erfindungsgemäßen Stoffe der Formel (I) vorzugsweise für $R^7$ genannt wurden, wobei Wasserstoff ausgenommen ist.

Die Verbindungen der Formel (V) sind allgemein bekannte Stoffe der organischen Chemie.

Das erfindungsgemäße Verfahren (c) wird unter den Bedingungen durchgeführt, die auch im Falle des erfindungsgemäßen Verfahrens (b) angewandt werden.

Die bei der Durchführung des erfindungsgemäßen Verfahrens (d) als Ausgangsstoffe benötigten Verbindungen sind durch die Formel (Ic) definiert. Diese Stoffe lassen sich nach den erfindungsgemäßen Verfahren (a), (b) und (c) herstellen.

Die erfindungsgemäße Reduktion gemäß Verfahren (d) erfolgt nach üblichen Methoden, z. B. durch Umsetzung von Verbindungen der Formel (Ic) mit komplexen Hydriden, gegebenenfalls in Gegenwart eines Verdünnungsmittels, oder mit Aluminiumisopropylat in Gegenwart eines Verdünnungsmittels.

Als komplexe Hydride kommen vorzugsweise Natriumborhydrid und Lithiumaluminiumhydrid in Frage.

Arbeitet man mit komplexen Hydriden, so kommen als Verdünnungsmittel für die erfindungsgemäße Umsetzung nach Verfahren (d) polare organische Lösungsmittel in Frage.

Vorzugsweise verwendbar sind Alkohole, wie Methanol, Ethanol, Butanol, Isopropanol und Ether, wie Diethylether oder Tetrahydrofuran.

Die Reduktion mit komplexen Hydriden wird in allgemeinen bei Temperatur zwischen 0 °C und 30 °C, vorzugsweise zwischen 0 °C und 20 °C, durchgeführt. Hierzu setzt man auf 1 Mol eines Ketons der Formel (Ic) etwa 1 Mol eines komplexen Hydrids, wie Natriumborhydrid oder Lithiumalanat, ein. Zur Isolierung der reduzierten Verbindungen der Formel (I) wird der Rückstand in verdünnter Säure, z. B. Salzsäure, aufgenommen, anschließend alkalisch gestellt und mit einem organischen Lösungsmittel extrahiert. Die weitere Aufarbeitung erfolgt in üblicher Weise.

Arbeitet man bei dem erfindungsgemäßen Verfahren (d) mit Aluminiumisopropylat, so kommen als Verdünnungsmittel für die Umsetzung bevorzugt Alkohole, wie Isopropanol, oder inerte Kohlenwasserstoffe, wie Benzol, in Frage. Die Reaktionstemperaturen können wiederum in einem größeren Bereich variiert werden. Im allgemeinen arbeitet man bei Temperaturen zwischen 20 °C und 120 °C, vorzugsweise zwischen 50 °C und 100 °C. Zur Durchführung der Reaktion setzt man auf 1 Mol eines Ketons der Formel (Ic) etwa 1 bis 2 Mol Aluminiumisopropylat ein. Zur Isolierung der reduzierten Verbindungen der Formel (I) verfährt man nach üblichen Methoden. Im allgemeinen geht man so vor, daß man das überschüssige Lösungsmittel durch Destillation unter vermindertem Druck entfernt, und das entstandene Produkte mit verdünnter Säure, wie Schwefelsäure, oder Base, wie wäßriger Natronlauge, behandelt. Die weitere Aufarbeitung erfolgt in üblicher Weise.

Bei der Durchführung der erfindungsgemäßen Verfahren (a) bis (d) arbeitet man im allgemeinen unter

Normaldruck. Es ist jedoch auch möglich, unter erhöhtem oder vermindertem Druck zu arbeiten.

Die bei der Durchführung des erfindungsgemäßen Verfahrens (e) als Ausgangsstoffe benötigten Verbindungen der Formel (Id) sind erfindungsgemäße Stoffe, die sich nach den Verfahren (a) bis (d) herstellen lassen.

Als Oxidationsmittel zur Durchführung des erfindungsgemäßen Verfahrens (e) kommen alle üblicherweise für Schwefeloxidationen verwendbaren Oxidationsmittel infrage. Vorzugsweise verwendet man Wasserstoffperoxid oder organische Persäuren, wie beispielsweise Peressigsäure, 4-Nitroperbenzoesäure oder 3-Chlorperbenzoesäure oder auch anorganische Oxidationsmittel, wie Periodsäure, Kaliumpermanganat oder Chromsäure.

Als Verdünnungsmittel zur Durchführung des erfindungsgemäßen Verfahrens (e) kommen in Abhängigkeit vom verwendeten Oxidationsmittel anorganische oder organische Lösungsmittel in Frage. Vorzugsweise verwendet man Alkohole, wie Methanol oder Ethanol oder deren Gemische mit Wasser sowie reines Wasser; Säuren, wie beispielsweise Essigsäure, Acetanhydrid oder Propionsäure oder dipolar aprotische Lösungsmittel, wie Acetonitril, Aceton, Essigsäureethylester oder Dimethylformamid sowie auch gegebenenfalls halogenierte Kohlenwasserstoffe, wie Benzin, Benzol, Toluol, Hexan, Cyclohexan, Petrolether, Dichlormethan, Dichlorethan, Chloroform, Tetrachlorkohlenstoff oder Chlorbenzol.

Das erfindungsgemäße Verfahren (e) kann gegebenenfalls in Gegenwart eines Säurebindemittels durchgeführt werden.

Als solche kommen alle üblicherweise verwendbaren organischen und anorganischen Säurebindemittel in Frage. Vorzugsweise verwendet man Erdalkali- oder Alkalimetallhydroxide, -acetate oder -carbonate, wie beispielsweise Calciumhydroxid, Natriumhydroxid, Natriumacetat oder Natriumcarbonat.

Das erfindungsgemäße Verfahren (e) kann gegebenenfalls in Gegenwart eines geeigneten Katalysators durchgeführt werden. Als solche kommen alle üblicherweise für derartige Schwefeloxidationen verwendbaren Katalysatoren in Frage. Vorzugsweise verwendet man Schwermetallkatalysatoren; beispielhaft genannt sei in diesem Zusammenhang Ammoniummolybdat.

Die Reaktionstemperaturen können bei der Durchführung des erfindungsgemäßen Verfahrens (e) in einem größeren Bereich variiert werden. Im allgemeinen arbeitet man bei Temperaturen zwischen -30 °C und +100 °C, vorzugsweise bei Temperaturen zwischen 0 °C und +80 °C.

Zur Durchführung des erfindungsgemäßen Verfahren (e) setzt man pro Mol der Verbindungen der Formel (Id) im allgemeinen 0,8 bis 1,2 Mol, vorzugsweise äquimolare Mengen an Oxidationsmittel ein, wenn man die Oxidation des Schwefels auf der Sulfoxidstufe unterbrechen will. Für eine Oxidation bis zur Sulfonstufe setzt man pro Mol der Verbindungen der Formel (Id) im allgemeinen 1,8 bis 3,0 Mol, vorzugsweise doppelt molare Mengen an Oxidationsmittel ein. Die Rekationsdurchführung, Aufarbeitung und Isolierung der Sulfinyl- bzw. Sulfonylverbindungen der Formel (I) erfolgt nach allgemein bekannten Verfahren.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z. B. durch Lösen einer Verbindung der allgemeinen Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe von Verbindungen der allgemeinen Formel (I) können in einfacher Weise nach üblichen Verfahren erhalten werden, so z. B. durch Lösen des Metallsalzes in Alkohol, z. B. Ethanol, und Hinzufügen zu Verbindungen der Formel (I). Man kann Metallsalz-Komplexe in bekannter Weise, z. B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;

Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten,wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg protektiv zur Bekämpfung von Cochliobolus-Arten bei Gerste, Sphaerotheca-Arten bei Gurken, Venturia-Arten bei Äpfeln und Pyricularia-Arten bei Reis sowie als Saatgutbehandlungsmittel bei Weizen gegen Fusarium culmorum einsetzen.

Außerdem besitzen die erfindungsgemäßen Wirkstoffe auch eine breite Wirkung gegen Getreidekrankheiten insbesondere gegen Mehltau, Rost, Pseudocercosporella herpotrichoides, Septoria nodorum und Pyrenophora teres als Spritzmittel sowie gegen Flugbrand, Streifenkrankheit und Mehltau als Saatbeizmittel. Ferner zeigen die erfindungsgemäßen Wirkstoffe auch eine gute Wirkung gegen Pellicularia sasakii an Reis im in-vitro-Test.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste

Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und Wachstumsgulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Herstellung und Verwendung der erfindungsgemäßen Wirkstoffe gehen aus den folgenden Beispielen hervor.

## Herstellungsbeispiele

### Beispiel 1

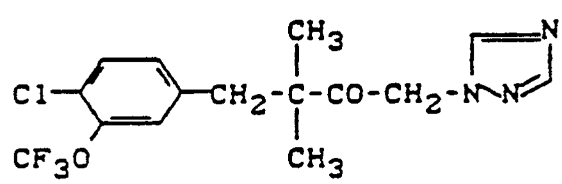

(Verfahren a)

10,3 g (0,15 Mol) 1,2,4-Triazol und 56,3 g (0,408 Mol) gemahlenes Kaliumcarbonat, werden in 300 ml Aceton vorgelegt. Bei 5 °C - 10 °C läßt man 50,8 g (0,136 Mol) 1-Brom-4-(4-chlor-3-trifluor-methoxy-phenyl)-3,3-dimethylbutan-2-on zutropfen. Nach Beendigung der Zugabe wird 8 Stunden bei Raumtemperatur gerührt. Zur Aufarbeitung wird das Reaktionsgemisch abgesaugt und das Filtrat unter vermindertem

Druck eingeengt. Der Rückstand wird in Methylenchlorid aufgenommen. Die organische Lösung wird mit Wasser gewachen, über Natriumsulfat getrocknet und unter vermindertem Druck eingeengt.

Man erhält 45,7 g (92,9 % der Theorie) 4-(4-Chlor-3-trifluormethoxy-phenyl)-3,3-dimethyl-1-(1,2,4-triazol-1-yl)-butan-2-on vom Brechungsindex $n_D^{20}$ = 1,5035.

Herstellung der Ausgangsprodukte

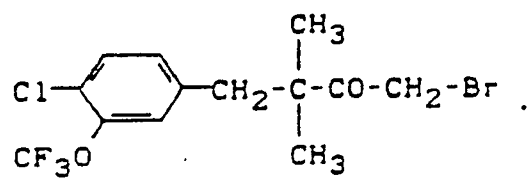

40 g (0,136 Mol) 4-(4-Chlor-3-trifluormethoxy-phenyl)-3,3-dimethyl-butan-2-on werden in 130 ml Chloroform gelöst. Bei Raumtemperatur läßt man unter Rühren 21,7 g (0,136 Mol) Brom zutropfen und nach Ende der Zugabe noch 1 Stunde nachrühren. Zur Aufarbeitung wird unter vermindertem Druck eingeengt.

Man erhält 50,8 g (100 % der Theorie) 1-Brom-4-(4-chlor-3-trifluormethoxy-phenyl)-3,3-dimethyl-butan-2-on vom Brechungsindex $n_D^{20}$ = 1,5015.

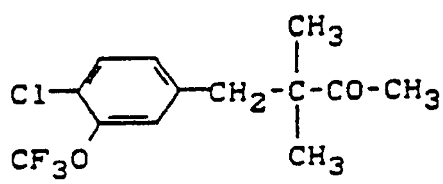

72,9 g (1,146 Mol) Kaliumhydroxid (gepulvert, 88%ig) und 3,8 g Tetrabutylammoniumbromid werden in 100 ml Toluol vorgelegt. Bei Raumtemperatur läßt man unter Rühren und unter Kühlung 39,4 g (0,458 Mol) Methylisopropylketon zutropfen. Danach läßt man bei 25 °C bis 30 °C unter Rühren 93,7 g (0,382 Mol) 4-Chlor-3-trifluormethoxy-benzylchlorid zutropfen und 8 Stunden bei Raumtemperatur nachrühren. Zur Aufarbeitung fügt man 200 ml Wasser zum Reaktionsgemisch und trennt die Phasen. Die organische Phase wird getrocknet und unter vermindertem Druck eingeengt. Der Rückstand wird einer fraktionierten Vakuumdestillation unterworfen.

Man erhält 35,8 g (31,8 % der Theorie) 4-(4-Chlor-3-trifluormethoxy-phenyl)-3,3-dimethyl-butan-2-on vom Siedepunkt 106 °C - 110 °C/0,1 mbar.

Beispiel 2

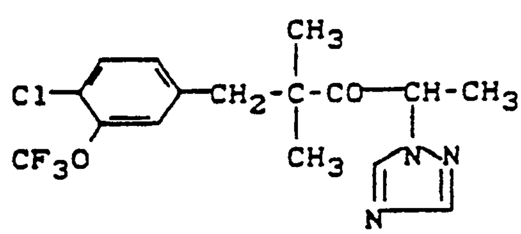

(Verfahren b)

Eine Lösung von 23 g (0,064 Mol) 4-(4-Chlor-3-trifluormethoxy-phenyl)-3,3-dimethyl-1-(1,2,4-triazol-1-yl)-butan-2-on und 3,6 g (0,064 Mol) Kaliumhydroxid in 12 ml Wasser und 100 ml Dimethylsulfoxid wird vorgelegt. Man gibt unter Rühren bei Raumtemperatur 9,1 g (0,064 Mol) Methyliodid in das Reaktionsgemisch, wobei die Temperatur bis auf 55 °C steigt. Danach läßt man noch 3 Stunden ohne Heizung

nachrühren. Zur Aufarbeitung wird das Reaktionsgemisch in 600 ml Wasser gegeben. Man extrahiert mit Methylenchlorid, wäscht die organische Phase zweimal mit Wasser, trocknet über Natriumsulfat und engt unter vermindertem Druck ein. Der Rückstand wird säulenchromatographisch an Kieselgel (Laufmittel Essigester/ Cyclohexan 3 : 1) gereinigt.

Man erhält 11,1 g (46,2 % der Theorie) 5-(4-Chlor-3-trifluormethoxy-phenyl)-4,4-dimethyl-2-(1,2,4-triazol-1-yl)-pentan-3-on vom Brechungsindex $n_D^{20}$ = 1,5004.

Beispiel 3

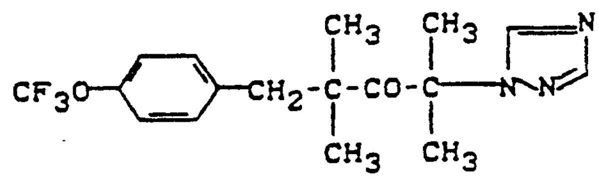

(Verfahren b)

235,4 g (0,72 Mol) 4-(4-Trifluormethoxy-phenyl)-3,3-dimethyl-1-(1,2,4-triazol-1-yl)-butan-2-on und 161,6 g (2,88 Mol) Kaliumhydroxid in 300 ml Wasser werden in 200 ml Dimethylsulfoxid vorgelegt. Bei 90 °C läßt man unter Rühren 306,5 g (2,16 Mol) Methyliodid in das Reaktionsgemisch tropfen und 3 Stunden bei 90 °C nachrühren. Danach wird das Reaktionsgemisch auf Raumtemperatur abgekühlt, in Wasser gegeben und mit Dichlormethan extrahiert. Die organische Phase wird über Natriumsulfat getrocknet und unter vermindertem Druck eingeengt. Der Rückstand wird säulenchromatographisch an Kieselgel (Laufmittel Essigester/Cyclohexan 3 : 1) gereinigt.

Man erhält 137,3 g (53,7 % der Theorie) 5-(4-Trifluormethoxyphenyl)-2,4,4-trimethyl-2-(1,2,4-triazol-1-yl)-pentan-3-on vom Schmelzpunkt 100 °C.

Beispiel 4

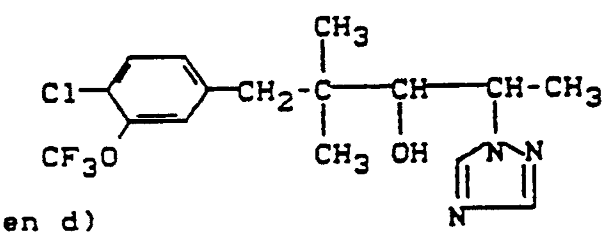

(Verfahren d)

10 g (0,027 Mol) 5-(4-Chlor-3-trifluormethoxy-phenyl)-4,4-dimethyl-2-(1,2,4-triazol-1-yl)-pentan-3-on werden in 100 ml Methanol vorgelegt. Bei Raumtemperatur läßt man eine Lösung von 0,27 g (0,0073 Mol) Natriumborhydrid, in 6 ml Wasser, zutropfen. Man läßt 2 Stunden bei Raumtemperatur nachrühren und engt das Reaktionsgemisch unter vermindertem Druck ein. Der Rückstand wird in Dichlormethan aufgenommen. Die organische Phase wird mit Wasser gewaschen, über Natriumsulfat getrocknet und unter vermindertem Druck eingeengt. Das Rohprodukt wird aus Petrolether umkristallisiert.

Man erhält 7,65 g (75 % der Theorie) 5-(4-Chlor-3-trifluormethoxy-phenyl)-4,4-dimethyl-2-(1,2,4-triazol-1-yl)-pentan-3-ol vom Schmelzpunkt 109 °C - 110 °C.

In analoger Weise zu den in den Beispielen 1 bis 4 beschreibenen Methoden und unter Berücksichtigung der Angaben zu den erfindungsgemäßen Verfahren (a) bis (e) werden die nachfolgend aufgeführten Verbindungen der Formel

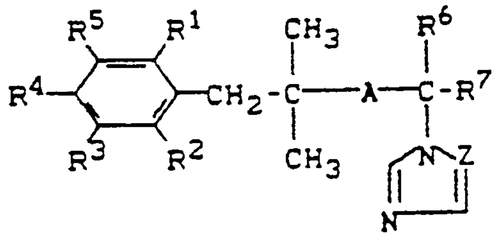

(I)

erhalten:

| Beisp.-Nr. | $R^1$ | $R^2$ | $R^3$ | $R^4$ | $R^5$ | $R^6$ | $R^7$ | Z | A | Brechungsindex Schmelzpunkt |
|---|---|---|---|---|---|---|---|---|---|---|
| 5 | H | H | H | $OCF_2Cl$ | H | H | H | N | -CO | $n_D^{20}$ = 1,5008 |
| 6 | H | H | H | $OCF_2Cl$ | H | H | $CH_3$ | N | -CO | $n_D^{20}$ = 1,505 |
| 7 | H | H | H | $OCF_2Cl$ | H | H | $CH_3$ | N | -CH-<br>OH | 82 °C |
| 8 | H | H | H | $OCF_3$ | Cl | H | H | N | -CO- | $n_D^{20}$ = 1,5004 |
| 9 | H | H | H | $OCF_3$ | Cl | H | $CH_3$ | N | -CO- | $n_D^{20}$ = 1,5001 |
| 10 | H | H | H | $OCF_3$ | Cl | H | $CH_3$ | N | -CH-<br>OH | 75° C - 77° C |
| 11 | H | H | H | $OCF_3$ | Cl | H | $-CH_2-CH=CH_2$ | N | -CO- | $n_D^{20}$ = 1,5000 |
| 12 | H | H | H | $OCF_3$ | Cl | H | $-CH_2-CH=CH_2$ | N | -CH-<br>OH | 68° C - 70° C |
| 13 | H | H | H | $OCF_3$ | H | $CH_3$ | $CH_3$ | N | -CH-<br>OH | 130 °C |

| Beisp.-Nr. | $R^1$ | $R^2$ | $R^3$ | $R^4$ | $R^5$ | $R^6$ | $R^7$ | Z | A | Brechungsindex Schmelzpunkt |
|---|---|---|---|---|---|---|---|---|---|---|
| 14 | H | H | Cl | $OCF_3$ | Cl | H | H | N | -CO- | $n_D^{20}$ = 1,5045 |
| 15 | H | H | Cl | $OCF_3$ | Cl | H | $CH_3$ | N | -CO- | $n_D^{20}$ = 1,5049 |
| 16 | H | H | Cl | $OCF_3$ | Cl | H | $CH_3$ | N | -CH-<br>OH | 83° C - 85° C |
| 17 | H | H | Cl | $OCF_2CHClF$ | H | H | H | N | -CO- | $n_D^{20}$ = 1,5029 |
| 18 | Cl | H | H | $SCF_3$ | H | H | H | N | -CO- | $n_D^{20}$ = 1,5228 |
| 19 | H | H | | 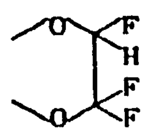 | H | H | H | N | -CO- | $n_D^{20}$ = 1,5083 |
| 20 | Cl | H | H | $SCF_3$ | H | H | $CH_3$ | N | -CO- | $n_D^{20}$ = 1,5229 |
| 21 | H | H | | 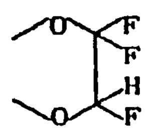 | H | H | $CH_3$ | N | -CO- | $n_D^{20}$ = 1,5002 |
| 22 | Cl | H | H | $SCF_3$ | H | H | $CH_3$ | N | -CH-<br>OH | $n_D^{20}$ = 1,5228 |

| Beisp.-Nr. | $R^1$ | $R^2$ | $R^3$ | $R^4$ | $R^5$ | $R^6$ | $R^7$ | Z | A | Brechungsindex Schmelzpunkt |
|---|---|---|---|---|---|---|---|---|---|---|
| 23 | H | H | | 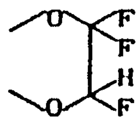 | H | H | $CH_3$ | N | -CH-<br>OH | 44° C - 46° C |
| 24 | H | H | H | $SCF_3$ | H | H | H | N | -CH-<br>OH | 78 °C |
| 25 | H | H | H | $OCF_3$ | H | H | H | N | -CH-<br>OH | 105° C - 107° C |
| 26 | H | H | H | $OCF_3$ | Cl | H | 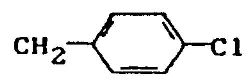 | N | -CO- | 114 °C |
| 27 | H | H | H | $OCF_3$ | Cl | H | $CH_2-CH=CH-CH_3$ | N | -CO- | $n_D^{20}$ = 1,5010 |
| 28 | H | H | H | $OCF_3$ | Cl | H | $CH_2-C\equiv CH$ | N | -CO- | $n_D^{20}$ = 1,5067 |
| 29 | H | H | H | $OCF_3$ | Cl | H | 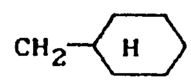 | N | -CO- | 92 °C |
| 30 | H | H | H | $OCHF_2$ | H | H | $CH_3$ | N | -CH-<br>OH | $n_D^{20}$ = 1,5072 |
| 31 | H | H | H | $OCF_3$ | Cl | H | 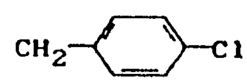 | N | -CH-<br>OH | 141 °C |

| Beisp.-Nr. | $R^1$ | $R^2$ | $R^3$ | $R^4$ | $R^5$ | $R^6$ | $R^7$ | Z | A | Brechungsindex Schmelzpunkt |
|---|---|---|---|---|---|---|---|---|---|---|
| 32 | H | H | H | $OCF_3$ | Cl | H | $-C_3H_7$ | N | -CO- | $n_D^{20}$ = 1,4845 |
| 33 | H | H | H | $OCHF_2$ | H | H | H | N | -CO- | $n_D^{20}$ = 1,5105 |
| 34 | H | H | H | $OCHF_2$ | H | H | $CH_3$ | N | -CO- | $n_D^{20}$ = 1,5081 |
| 35 | H | H | H | $OCF_3$ | Cl | H | $-C_3H_7$ | N | -CH(OH)- | 108° C |
| 36 | H | H | H | $OCF_3$ | Cl | H | $CH_2$- 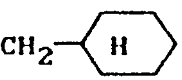 | N | -CH(OH)- | 129° C |
| 37 | H | H | H | $OCF_3$ | Cl | H | $CH_2-CH=CH-CH_3$ | N | -CH(OH)- | 88° C |
| 38 | H | H | H | $OCF_3$ | Cl | H | $CH_2-C\equiv CH$ | N | -CH(OH)- | 112° C |
| 39 | H | H | H | $OCF_3$ | Cl | $CH_3$ | $CH_2$- 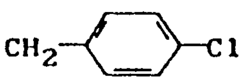 -Cl | N | -CO- | 110° C |
| 40 | H | H | H | $OCF_3$ | Cl | $CH_3$ | $CH_2$- 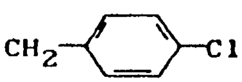 -Cl | N | -CH(OH)- | 80° C |

Verwendungsbeispiele

In den folgenden Verwendungsbeispielen wurden die nachstehend angegebenen Verbindungen als Vergleichssubstanzen eingesetzt:

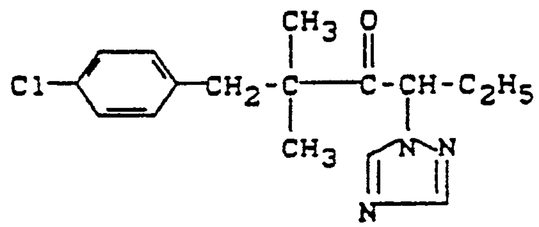

(A)

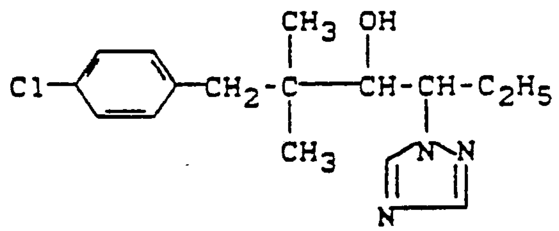

(B)

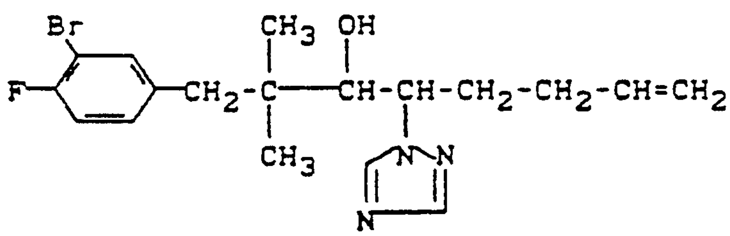

(C)

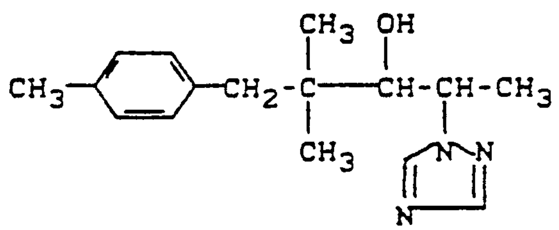

(D)

(Die Vergleichssubstanzen (A), (B), (C) und (D) sind bekannt aus US-A 4 549 900).

Beispiel A

Cochliobolus sativus-Test (Gerste)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

In diesem Test zeigen die in den Beispielen (7), (10), (12) und (13) aufgeführten erfindungsgemäßen Verbindungen eine wesentlich bessere Wirksamkeit als die Vergleichssubstanz (A).

Beispiel B

Fusarium culmorum-Test (Weizen) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Weizen sät man mit 2 x 100 Korn 1 cm tief in eine Standarderde und kultiviert ihn im Gewächshaus bei einer Temperatur von ca. 18 °C in Saatkästen, die täglich 15 Stunden dem Licht ausgesetzt werden.

Ca. 3 Wochen nach der Aussaat erfolgt die Auswertung der Pflanzen auf Symptome.

In diesem Test zeigt die in dem Beispiel (7) aufgeführte erfindungsgemäße Verbindung eine wesentlich bessere Wirksamkeit als die Vergleichssubstanz (B).

Beispiel C

Sphaerotheca-Test (Gurke) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24 °C und bei einer relativen Luftfeuchtigkeit von ca. 75 % im Gewächshaus aufgestellt.

10 Tage nach der Inokulation erfolgt die Auswertung.

In diesem Test zeigen die in den Beispielen (7), (10) und (12) aufgeführten erfindungsgemäßen Verbindungen eine wesentlich bessere Wirksamkeit als die Vergleichssubstanzen (A) und (D).

Beispiel D

Venturia-Test (Apfel) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

In diesem Test zeigen die in den Beispielen (7), (10), (12) und (13) aufgeführten erfindungsgemäßen Verbindungen eine wesentlich bessere Wirksamkeit als die Vergleichssubstanzen (B) und (C).

Beispiel E

Pyricularia-Test (Reis) /protektiv

Lösungsmittel: 12,5 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

In diesem Test zeigen die in den Beispielen (7), (10), (12) und (13) aufgeführten erfindungsgemäßen Verbindungen eine wesentlich bessere Wirksamkeit als die Vergleichssubstanzen (A) und (D).

**Patentansprüche**

1. 1,4-Disubstituierte 1-Azolyl-3,3-dimethylbutanDerivate der Formel

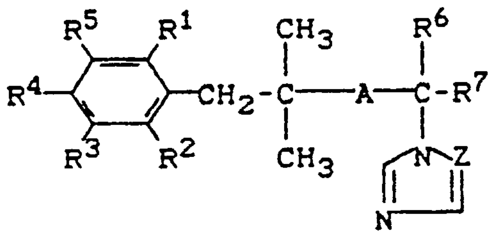

(I)

in welcher

| | |
|---|---|
| $R^1$ | für Wasserstoff, Fluor, Chlor oder Brom steht, |
| $R^2$ | für Wasserstoff, Fluor, Chlor oder Brom steht, |
| $R^3$ | für Wasserstoff, Fluor, Chlor, Brom oder Trifluormethoxy steht, |
| $R^4$ | für Fluor, Chlor, Brom, Trifluormethoxy, Trifluormethylthio, Trifluormethylsulfinyl, Trifluormethylsulfonyl, Difluormethoxy, Difluorchlormethoxy, Trifluorchlorethoxy, Tetrafluorethoxy, Trifluorethoxy, Difluormethylthio, Trifluorchlorethylthio, Tetrafluorethylthio, Difluormethylsulfinyl, Difluorchlormethylsulfinyl, Trifluorchlorethylsulfinyl, Tetrafluorethylsulfinyl, Difluormethylsulfonyl, Difluorchlormethylsulfonyl, Trifluorchlorethylsulfonyl, Tetrafluorethylsulfonyl, Difluorbrommethylthio, Difluorbrommethylsulfinyl, Difluorbrommethylsulfonyl oder Difluorchlormethylthio steht oder |
| $R^3$ und $R^4$ | gemeinsam für eine Gruppierung der Formel |

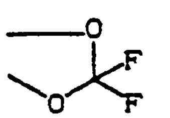

,

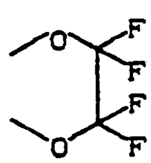

oder

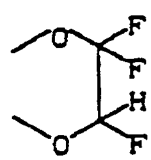

stehen,

| | |
|---|---|
| A | für eine Ketogruppe oder eine CH(OH)-Gruppierung steht, |
| Z | für ein Stickstoffatom oder die CH-Gruppe steht und |
| $R^6$ und $R^7$ | gleich oder verschieden sind und unabhängig voneinander für Wasserstoff, geradkettiges oder verzweigtes Alkyl mit 1 bis 12 Kohlenstoffatomen; geradkettiges oder verzweigtes Alkenyl oder Alkinyl mit jeweils 3 bis 12 Kohlenstoffatomen; gegebenenfalls einfach oder zweifach, gleichartig oder verschieden durch Halogen und/oder Alkyl substituiertes Cycloalkyl mit 3 bis 7 Kohlenstoffatomen; gegebenenfalls einfach oder zweifach, gleichartig oder verschieden durch Halogen und/oder Alkyl substituiertes Cycloalkylalkyl mit 3 bis 7 Kohlenstoffatomen im Cycloalkylteil und 1 bis 4 Kohlenstoffatomen im Alkylteil stehen oder für gegebenenfalls substituiertes |

Phenylalkyl mit 1 bis 4 Kohlen-stoffatomen im Alkylteil stehen, wobei als Phenylsubstituenten Halogen, Alkyl mit 1 bis 4 Kohlenstoffatomen; Alkoxy und Alkylthio mit jeweils 1 bis 4 Kohlenstoffatomen; Halogenalkyl, Halogenalkoxy, Halogenalkylthio, Halogenalkylsulfinyl und Halogenalkylsulfonyl mit jeweils 1 bis 4 Kohlenstoffatomen und 1 bis 5 gleichen oder verschiedenen Halogenatomen, wie Fluor-, Chlor- und/oder Bromatomen; Nitro, Cyano, Alkoxycarbonyl mit 1 bis 4 Kohlenstoffatomen im Alkoxyteil oder gegebenenfalls einfach bis dreifach, gleich oder verschieden durch Fluor, Chlor und/oder Brom substituiertes Phenyl und Phenoxy genannt seien oder zwei benachbarte Phenylsubstituenten gemeinsam für eine Gruppierung der Formel

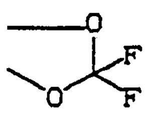 , 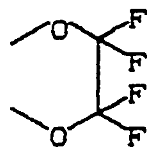 oder 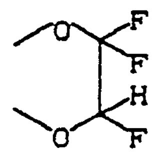

stehen,
wobei jedoch $R^3$ für Trifluormethoxy steht, wenn $R^4$ für Fluor, Chlor oder Brom steht,

sowie deren Säureadditionssalze und Metallsalzkomplexe.

2. 1,4-Disubstituiertes 1-Azolyl-3,3-dimethyl-butanDerivat gemäß Anspruch 1, gekennzeichnet durch die Formel

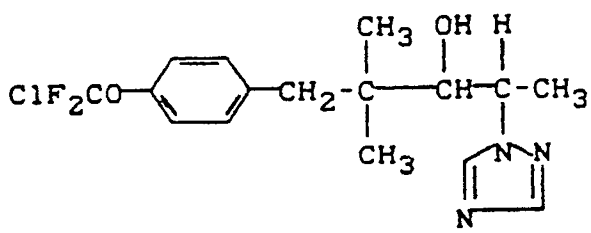

3. 1,4-Disubstituiertes 1-Azolyl-3,3-dimethyl-butanDerivat gemäß Anspruch 1, gekennzeichnet durch die Formel

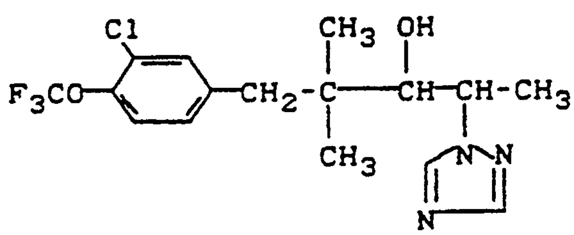 .

4. 1,4-Disubstituiertes 1-Azolyl-3,3-dimethyl-butanDerivat gemäß Anspruch 1, gekennzeichnet durch die Formel

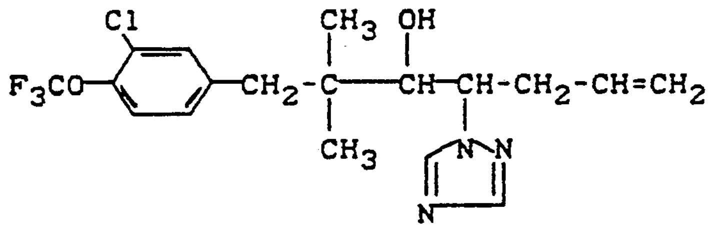

5. Verfahren zur Herstellung von 1,4-disubstituierten 1-Azolyl-3,3-dimethylbutan-Derivaten der Formel

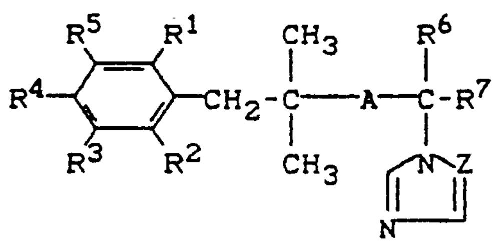

(I)

in welcher

$R^1$ $R^2$ $R^3$ $R^4$ $R^5$ $R^6$ $R^7$ A und Z die oben angegebene Bedeutung haben,

sowie von deren Säureadditionssalzen und Metallsalzkomplexen,

**dadurch gekennzeichnet,** daß man

(a) Halogen-ketone der Formel

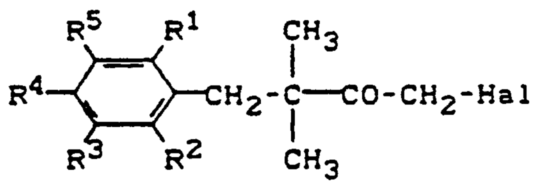

(II)

in welcher

$R^1$, $R^2$, $R^3$, $R^4$, $R^5$ die oben angegebene Bedeutung haben und

Hal für Chlor, Brom oder Iod steht,

mit einem Azol der Formel

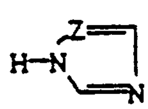

(III)

in welcher

Z die oben angegebene Bedeutung hat,

in Gegenwart eines Verdünnungsmittels und in Gegenwart eines Säurebindemittels umsetzt,

oder

(b) Ketone der Formel

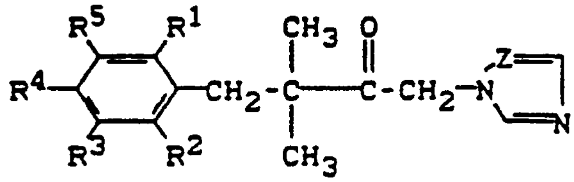

(Ia)

in welcher

$R^1$, $R^2$, $R^3$, $R^4$, $R^5$ und Z die oben angegebene Bedeudeutung haben,

mit einer Verbindung der Formel

$R^8$ - Y (IV)

in welcher

$R^8$ für geradkettiges oder verzweigtes Alkyl mit 1 bis 12 Kohlenstoffatomen; geradkettiges oder verzweigtes Alkenyl oder Alkinyl mit jeweils 3 bis 12 Kohlenstoffatomen; gegebenenfalls einfach oder zweifach, gleichartig oder verschieden durch Halogen und/oder Alkyl substituiertes Cycloalkyl mit 3 bis 7 Kohlenstoffatomen; gegebenenfalls einfach oder zweifach, gleichartig oder verschieden durch Halogen und/oder Alkyl substituiertes Cycloalkylalkyl mit 3 bis 7 Kohlenstoffatomen im Cycloalkylteil und 1 bis 4 Kohlenstoffatomen im Alkylteil steht oder für gegebenenfalls substituiertes Phenylalkyl mit 1 bis 4 Kohlenstoffatomen im Alkylteil steht, wobei als Phenylsubstituenten Halogen, Alkyl mit 1 bis 4 Kohlenstoffatomen; Alkoxy und Alkylthio mit jeweils 1 bis 4 Kohlenstoffatomen; Halogenalkyl, Halogenalkoxy, Halogenalkylthio, Halogenalkylsulfinyl und Halogenalkylsulfonyl mit jeweils 1 bis 4 Kohlenstoffatomen und 1 bis 5 gleichen oder verschiedenen Halogenatomen, wie Fluor-, Chlor- und/oder Bromatomen; Nitro, Cyano, Alkoxycarbonyl mit 1 bis 4 Kohlenstoffatomen im Alkoxyteil oder gegebenenfalls einfach bis dreifach, gleich oder verschieden durch Fluor, Chlor und/oder Brom substituiertes Phenyl und Phenoxy genannt seien oder zwei benachbarte Phenylsubstituenten gemeinsam für eine Gruppierung der Formel

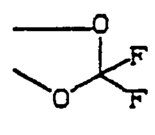

,

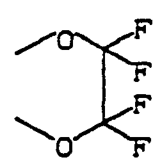

oder

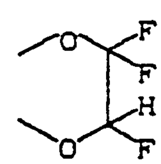

stehen,
und

Y für Chlor, Brom, Iod, p-Methylphenylsulfonyloxy, die Gruppierung $-O-SO_2-OR$ oder $NR_3$ steht, wobei R für Methyl, Ethyl, n- oder i-Propyl steht,

gegebenenfalls in Gegenwart eines Säurebindemittels sowie in Gegenwart eines Verdünnungsmittels und gegebenenfalls in Gegenwart eines Katalysators umsetzt,

oder

(c) Ketone der Formel

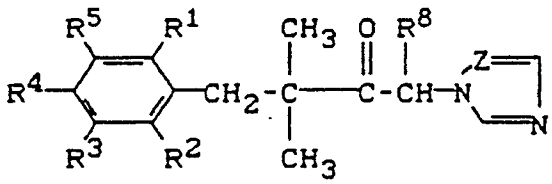

(Ib)

in welcher

$R^1$, $R^2$, $R^3$, $R^4$, $R^5$, $R^8$ und Z die oben angegebene Bedeutung haben,

mit einer Verbindung der Formel

$R^9$ - $Y^1$ (V)

in welcher

$R^9$ für geradkettiges oder verzweigtes Alkyl mit 1 bis 12 Kohlenstoffatomen; geradkettiges oder verzweigtes Alkenyl oder Alkinyl mit jeweils 3 bis 12 Kohlenstoffatomen; gegebenenfalls einfach oder zweifach, gleichartig oder verschieden durch Halogen und/oder Alkyl substituiertes Cycloalkyl mit 3 bis 7 Kohlenstoffatomen; gegebenenfalls einfach oder zweifach, gleichartig oder verschieden durch Halogen und/oder Alkyl substituiertes Cycloalkylalkyl mit 3 bis 7 Kohlenstoffatomen im Cycloalkylteil und 1 bis 4 Kohlenstoffatomen im Alkylteil steht oder für gegebenenfalls substituiertes Phenylalkyl mit 1 bis 4 Kohlenstoffatomen im Alkylteil steht, wobei als Phenylsubstituenten Halogen, Alkyl mit 1 bis 4 Kohlenstoffatomen; Alkoxy und Alkylthio mit jeweils 1 bis 4 Kohlenstoffatomen; Halogenalkyl, Halogenalkoxy, Halogenalkylthio, Halogenalkylsulfinyl und Halogenalkylsulfonyl mit jeweils 1 bis 4 Kohlenstoffatomen und 1 bis 5 gleichen oder verschiedenen Halogenatomen, wie Fluor-, Chlor- und/oder Bromatomen; Nitro, Cyano, Alkoxycarbonyl mit 1 bis 4 Kohlenstoffatomen im Alkoxyteil oder gegebenenfalls einfach bis dreifach, gleich oder verschieden durch Fluor, Chlor und/oder Brom substituiertes Phenyl und Phenoxy genannt seien oder zwei benachbarte Phenylsubstituenten gemeinsam für eine Gruppierung der Formel

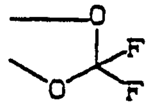

,

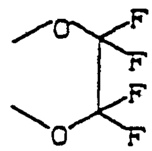

oder

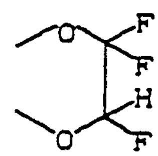

stehen,
und

$Y^1$ für Chlor, Brom, Iod, p-Methylphenylsulfonyloxy, die Gruppierung $-O-SO_2-OR$ oder $NR_3$ steht, wobei R für Methyl, Ethyl, n- oder i-Propyl steht,

gegebenenfalls in Gegenwart eines Säurebindemittels sowie in Gegenwart eines Verdünnungsmittels und gegebenenfalls in Gegenwart eines Katalysators umsetzt,

oder

(d) Ketone der Formel

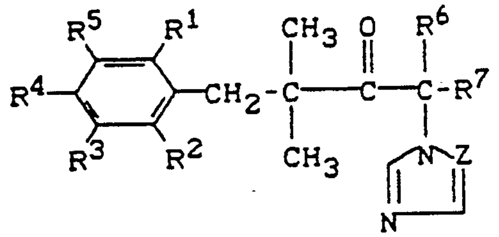

(Ic)

in welcher

$R^1$, $R^2$, $R^3$, $R^4$, $R^5$, $R^6$ und $R^7$ die oben angegebene Bedeutung haben,

mit einem Reduktionsmittel gegebenenfalls in Gegenwart eines Verdünnungsmittels umsetzt,

oder

(e) 1,4-disubstituierte 1-Azolyl-3,3-dimethylbutan-Derivate der Formel

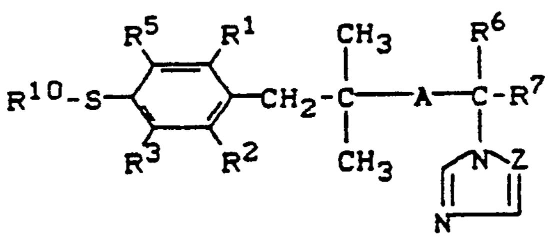

(Id)

in welcher

$R^1$, $R^2$, $R^3$, $R^5$, $R^6$, $R^7$, A und Z die oben angegebene Bedeutung haben und
$R^{10}$ für Trifluormethyl, Difluormethyl, Trifluorchlorethyl, Tetrafluorethyl, Difluorchlormethyl oder Difluorbrommethyl steht,

mit einem Oxidationsmittel, gegebenenfalls in Gegenwart eines Verdünnungsmittels und gegebenenfalls in Gegenwart eines Reaktionshilfsmittels umsetzt,

und gegebenenfalls an die so erhaltenen Verbindungen der Formel (I) eine Säure oder ein Metallsalz addiert.

6. Fungizide Mittel, gekennzeichnet, durch einen Gehalt an mindestens einem 1,4-disubstituierten 1-Azolyl-3,3-dimethyl-butan-Derivat der Formel (I) gemäß Anspruch 1 bzw. einem Säureadditions-Salz oder Metallsalz-Komplex eines 1,4-disubstituierten 1-Azolyl-3,3-dimethyl-butan-Derivates der Formel (I).

7. Verwendung von 1,4-disubstituierten 1-Azolyl-3,3-dimethyl-butan-Derivatender Formel (I) gemäß Anspruch 1 bzw. von deren Säureadditions-Salzen oder Metallsalz-Komplexen zur Bekämpfung von Pilzen.

8. Verfahren zur Bekämpfung von Pilzen, dadurch gekennzeichnet, daß man 1,4-disubstituierte 1-Azolyl-3,3-dimethyl-butan-Derivate der Formel (I) gemäß Anspruch 1 bzw. deren Säureadditions-Salze oder Metallsalz-Komplexe auf Pilze und/oder ihren Lebensraum ausbringt.

9. Verfahren zur Herstellung von fungiziden Mitteln, dadurch gekennzeichnet, daß man 1,4-disubstituierte 1-Azolyl-3,3-dimethyl-butan-Derivate der Formel (I) gemäß Anspruch 1 bzw. deren Säureadditions-Salze oder Metallsalz-Komplexe mit Streckmitteln und/oder oberflächenaktiven Stoffen vermischt.

## Claims

1. 1,4-Disubstituted 1-azolyl-3,3-dimethylbutane derivatives of the formula

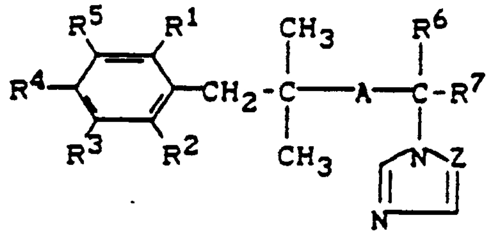

(I)

in which

| | |
|---|---|
| $R^1$ | represents hydrogen, fluorine, chlorine or bromine, |
| $R^2$ | represents hydrogen, fluorine, chlorine or bromine, |
| $R^3$ | represents hydrogen, fluorine, chlorine, bromine or trifluoromethoxy, |
| $R^4$ | represents fluorine, chlorine, bromine, trifluoromethoxy, trifluoromethylthio, trifluoromethylsulphinyl, trifluoromethylsulphonyl, difluoromethoxy, difluorochloromethoxy, trifluorochloroethoxy, tetrafluoroethoxy, trifluoroethoxy, difluoromethylthio, trifluorochloroethylthio, tetrafluoroethylthio, difluoromethylsulphinyl, difluorochloromethylsulphinyl, trifluorochloroethylsulphinyl, tetrafluoroethylsulphinyl, difluoromethylsulphonyl, difluorochloromethylsulphonyl, trifluorochloroethylsulphonyl, tetrafluoroethylsulphonyl, difluorobromomethylthio, difluorobromomethylsulphinyl, difluorobromomethylsulphonyl or difluorochloromethylthio or |
| $R^3$ and $R^4$ | together represent a grouping of the formula |

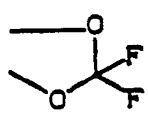 , 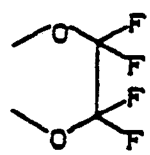 or 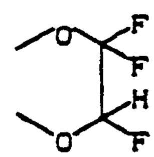 ,

| | |
|---|---|
| A | represents a keto group or a CH(OH) grouping, |
| Z | represents a nitrogen atom or the CH group and |
| $R^6$ and $R^7$ | are identical or different and independently of one another represent hydrogen, straight-chain or branched alkyl having 1 to 12 carbon atoms; straight-chain or branched alkenyl or alkinyl each having 3 to 12 carbon atoms; cycloalkyl having 3 to 7 carbon atoms which is optionally monosubstituted or disubstituted by identical or different halogen and/or alkyl substituents; cycloalkylalkyl which has 3 to 7 carbon atoms in the cycloalkl moiety and 1 to 4 carbon atoms in the alkyl moiety and is optionally monosubstituted or disubstituted by identical or different halogen and/or alkyl substituents, or represent optionally substituted phenylalkyl having 1 to 4 carbon atoms in the alkyl moiety, where phenyl substituents which may be mentioned are halogen, alkyl having 1 to 4 carbon atoms; alkoxy and alkylthio each having 1 to 4 carbon atoms; halogenoalkyl, halogenoalkoxy, halogenoalkylthio, halogenoalkylsulphinyl and halogenoalkylsulphonyl each having 1 to 4 carbon atoms and 1 to 5 identical or different halogen atoms, such as fluorine, chlorine and/or bromine atoms; nitro, cyano, alkoxycarbonyl having 1 to 4 carbon atoms in the alkoxy moiety, or phenyl and phenoxy which are both optionally monosubstituted to trisubstituted by identical or different fluorine, chlorine and/or bromine substituents, or two adjacent phenyl substituents together represent a grouping of the formula |

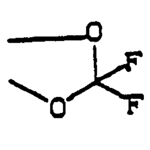 , 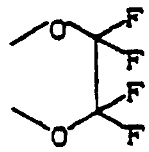 or 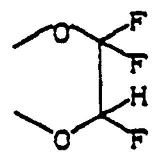 ,

where $R^3$ represents trifluoromethoxy, however, when $R^4$ represents fluorine, chlorine or bromine and also their acid addition salts and metal salts complexes.

2. 1,4-Disubstituted 1-azolyl-3,3-dimethyl-butane derivative according to Claim 1, characterised by the formula

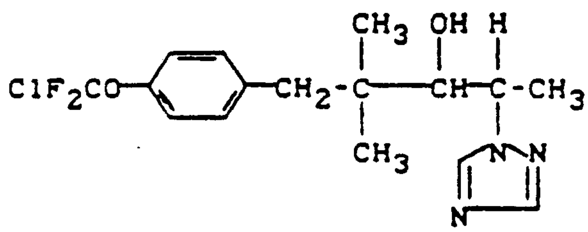 .

3. 1,4-Disubstituted 1-azolyl-3,3-dimethyl-butane derivative according to Claim 1, characterised by the formula

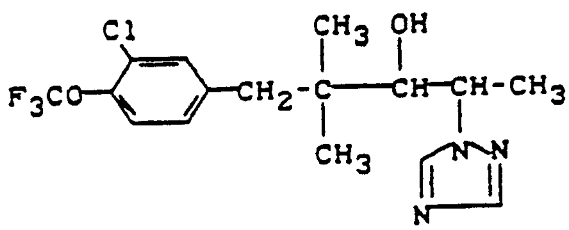 .

4. 1,4-Disubstituted 1-azolyl-3,3-dimethyl-butane derivative according to Claim 1, characterised by the formula

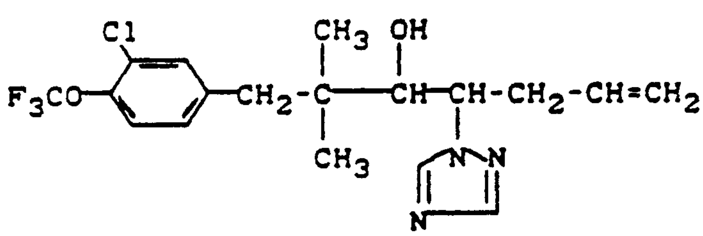 .

5. Process for the preparation of 1,4-disubstituted 1-asolyl-3,3-dimethylbutane derivatives of the formula

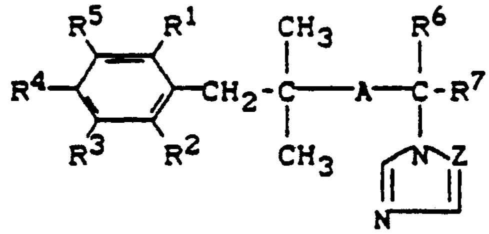

(I)

in which

$R^1$, $R^2$, $R^3$, $R^4$, $R^5$, $R^6$, $R^7$, A and Z have the abovementioned meaning, and also of their acid addition salts and metal salt complexes, characterised in that

(a) halogeno-ketones of the formula

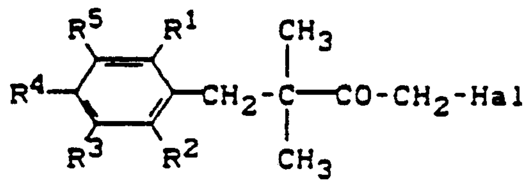

(II)

in which

$R^1$, $R^2$, $R^3$, $R^4$, and $R^5$ have the abovementioned meaning and

Hal represents chlorine, bromine or iodine,

are reacted with an azole of the formula

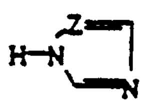

(III)

in which

Z has the abovementioned meaning,

in the presence of a diluent and in the presence of an acid-binding agent,

or

(b) ketones of the formula

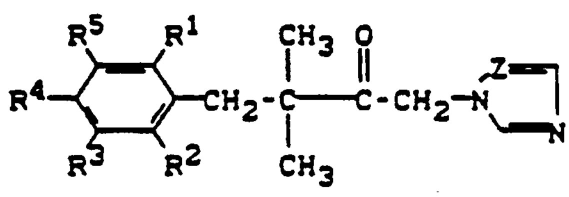

(Ia)

in which

$R^1$, $R^2$, $R^3$, $R^4$, $R^5$ and Z have the abovementioned meaning, are reacted with a compound of the formula

$R^8$ - Y (IV)

in which

$R^8$ represents straight-chain or branched alkyl having 1 to 12 carbon atoms; straight-chain or branched alkenyl or alkinyl each having 3 to 12 carbon atoms; cycloalkyl having 3 to 7 carbon atoms which is optionally monosubstituted or disubstituted by identical or different halogen and/or alkyl substituents; cycloalkylalkyl which has 3 to 7 carbon atoms in the cycloalkyl moiety and 1 to 4 carbon atoms in the alkyl moiety and is optionally monosubstituted or disubstituted by identical or different halogen and/or alkyl substituents, or represents optionally substituted phenylalkyl having 1 to 4 carbon atoms in the alkyl moiety, where phenyl substituents which may be mentioned are halogen, alkyl having 1 to 4 carbon atoms; alkoxy and alkylthio each having 1 to 4 carbon atoms; halogenoalkyl, halogenoalkoxy, halogenoalkylthio, halogenoalkylsulphinyl and halogenoalkylsulphonyl each having 1 to 4 carbon atoms and 1 to 5 identical or different halogen atoms, such as fluorine, chlorine and/or bromine atoms; nitro, cyano, alkoxycarbonyl having 1 to 4 carbon atoms in the alkoxy moiety, or phenyl and phenoxy which are both optionally monosubstituted to trisubstituted by identical or different fluorine, chlorine and/or bromine substituents, or two adjacent phenyl substituents together represents a grouping of the formula

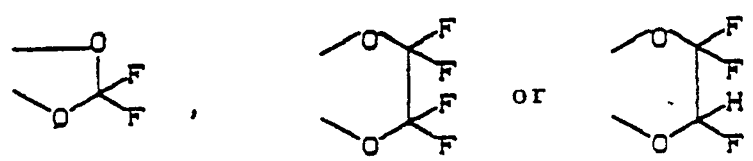

and

Y represents chlorine, bromine, iodine, p-methylphenylsulphonyloxy, the grouping $-O-SO_2-OR$ or $NR_3$, where R represents methyl, ethyl, n- or i-propyl,

if appropriate in the presence of an acid-binding agent and also in the presence of a diluent and if appropriate in the presence of a catalyst,

or

(c) ketones of the formula

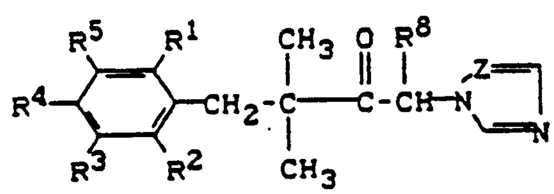

(Ib)

in which

$R^1$, $R^2$, $R^3$, $R^4$, $R^5$, $R^8$ and Z have the abovementioned meaning,

are reacted with a compound of the formula

$R^9$ - $Y^1$ (V)

in which

$R^9$ represents straight-chain or branched alkyl having 1 to 12 carbon atoms; straight-chain or branched alkenyl or alkinyl each having 3 to 12 carbon atoms; cycloalkyl having 3 to 7 carbon atoms which is optionally monosubstituted or disubstituted by identical or different halogen and/or alkyl sub-stituents; cycloalkylalkyl which has 3 to 7 carbon atoms in the cycloalkyl moiety and 1 to 4 carbon atoms in the alkyl moiety and is optionally monosubstituted or disubstituted by identical or different halogen and/or alkyl substituents, or represent optionally substituted phenylalkyl having 1 to 4 carbon atoms in the alkyl moiety, where phenyl substituents which may be mentioned are halogen, alkyl having 1 to 4 carbon atoms; alkoxy and alkylthio each having 1 to 4 carbon atoms; halogenoalkyl, halogenoalkoxy, halogenoalkylthio, halogenoalkylsulphinyl and halogenoalkylsulphonyl each having 1 to 4 carbon atoms and 1 to 5 identical or different halogen atoms, such as fluorine, chlorine and/or bromine atoms; nitro, cyano, alkoxycarbonyl having 1 to 4 carbon atoms in the alkoxy moiety, or phenyl and phenoxy which are both optionally monosubstituted to trisubstituted by identical or different fluorine, chlorine and/or bromine substituents, or two adjacent phenyl substituents together represent a grouping of the formula

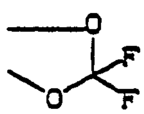 , 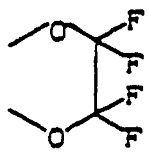 or 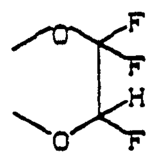 ,

$Y^1$ represents chlorine, bromine, iodine, p-methylphenylsulphonyloxy, the grouping $-O-SO_2-OR$ or $NR_3$, where R represents methyl, ethyl, n- or i-propyl,

if appropriate in the presence of an acid-binding agent and also in the presence of a diluent and if appropriate in the presence of a catalyst,

or

(d) ketones of the formula

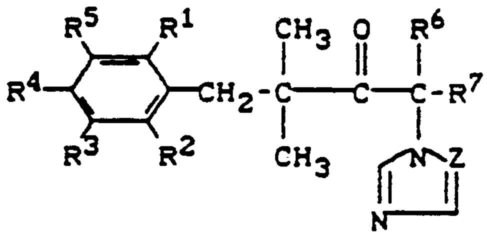 (Ic)

in which

$R^1$, $R^2$, $R^3$, $R^4$, $R^5$, $R^6$ and $R^7$ have the abovementioned meaning,
are reacted with a reductant if appropriate in the presence of a diluent,

or

(e) 1,4-disubstituted 1-azolyl-3,3-dimethyl-butane derivatives of the formula

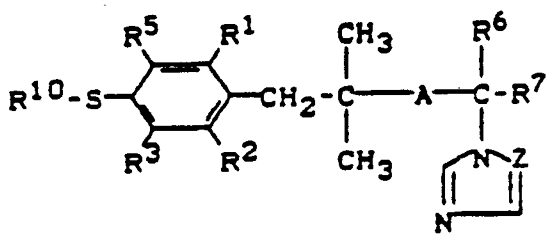

(Id)

in which

$R^1$, $R^2$, $R^3$, $R^5$, $R^6$ $R^7$ A and Z have the abovementioned meaning and

$R^{10}$ represents trifluoromethyl, difluoromethyl, trifluorochloroethyl, tetrafluoroethyl, difluorochloromethyl or difluorobromomethyl,

are reacted with an oxidant, if appropriate in the presence of a diluent and if appropriate in the presence of a reaction auxiliary, and if appropriate an acid or a metal salt is added to the compounds of the formula (I) thus obtained.

6. Fungicides, characterised in that they contain at least one 1,4-disubstituted 1-azolyl-3,3-dimethyl-butane derivative of the formula (I) according to Claim 1 or one acid addition salt or metal salt complex of a 1,4-disubstituted 1-azolyl-3,3-dimethyl-butane derivative of the formula (I).

7. Use of 1,4-disubstituted 1-azolyl-3,3-dimethylbutane derivatives of the formula (I) according to Claim 1 or of their acid addition salts or metal salt complexes for combating fungi.

8. Method of combating fungi, characterised in that 1,4-disubstituted 1-azolyl-3,3-dimethyl-butane derivatives of the formula (I) according to Claim 1 or their acid addition salts or metal salt complexes are applied to the fungi and/or their environment.

9. Process for the production of fungicides, characterised in that 1,4-disubstituted 1-azolyl-3, 3-dimethylbutane derivatives of the formula (I) according to Claim 1 or their acid addition salts or metal salt complexes are mixed with extenders and/or surface-active substances.

**Revendications**

1. Dérivés 1,4-disubstitués du 1-azolyl-3,3-diméthylbutane, de formule

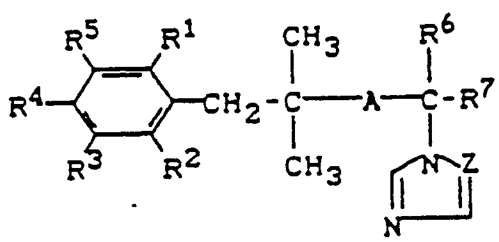

(I)

dans laquelle
$R^1$ représente l'hydrogène, le fluor, le chlore ou le brome,
$R^2$ représente l'hydrogène, le fluor, le chlore ou le brome,
$R^3$ représente l'hydrogène, le fluor, le chlore, le brome ou un groupe trifluorométhoxy,
$R^4$ représente le fluor, le chlore, le brome, un groupe trifluorométhoxy, trifluorométhylthio, trifluorométhylsulfinyle, trifluorométhylsulfonyle, difluorométhoxy, difluorochlorométhoxy, trifluorochloréthoxy, tétrafluoréthoxy, trifluoréthoxy, difluorométhylthio, trifluorochloréthylthio, tétrafluoréthylthio, difluorométhylsulfinyle, difluorochlorométhylsulfinyle, trifluorochloréthylsulfinyle, tétrafluoréthylsulfinyle, difluorométhylsulfonyle, difluorochlorométhylsulfonyle,trifluorochloréthylsulfonyle, tétrafluoréthylsulfonyle, difluorobromométhylthio, difluorobromométhylsulfinyle, difluorobromométhylsulfonyle ou difluorochlorométhylthio, ou bien

$R^3$ et $R^4$ forment ensemble un groupement de formule

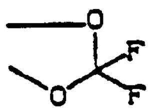 , 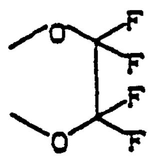 ou 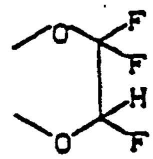

A représente un groupe céto ou un groupe CH(OH),

Z représente un atome d'azote ou le groupe CH, et

$R^6$ et $R^7$, ayant des significations identiques ou différentes, représentent chacun, indépendamment l'un de l'autre, l'hydrogène, un groupe alkyle à chaîne droite ou ramifiée en $C_1$-$C_{12}$; un groupe alcényle ou alcynyle à chaîne droite ou ramifiée contenant chacun 3 à 12 atomes de carbone;

un groupe cycloalkyle en $C_3$-$C_7$ portant éventuellement un ou deux substituants identiques ou différents choisis parmi les halogènes et les groupes alkyle; un groupe cycloakylalkyle contenant 3 à 7 atomes de carbone dans la partie cycloalkyle et 1 à 4 atomes de carbone dans la partie alkyle et portant éventuellement un ou deux substituants identiques ou différents choisis parmi les halogènes et les groupes alkyle, ou un groupe phénylalkyle éventuellement substitué contenant 1 à 4 atomes de carbone dans la partie alkyle, les substituants du noyau phényle étant des halogènes, des groupes alkyle en $C_1$-$C_4$; un groupe alcoxy ou alkylthio contenant chacun 1 à 4 atomes de carbone; un groupe halogénoalkyle, halogénoalcoxy, halogénoalkylthio, halogénoalkylsulfinyle ou halogénoalkylsulfonyle contenant chacun 1 à 4 atomes de carbone et 1 à 5 atomes d'halogènes identiques ou différents, par exemple des atomes de fluor, de chlore et/ou de brome ; un groupe nitro, cyano, alcoxycarbonyle contenant 1 à 4 atomes de carbone dans la partie alcoxy ou un groupe phényle ou phénoxy portant éventuellement un à trois substituants identiques ou différents choisis parmi le fluor, le chlore et le brome, ou bien deux substituants phényle voisins forment ensemble un groupement de formule

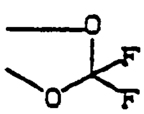 , 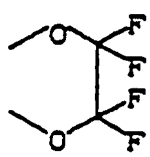 ou 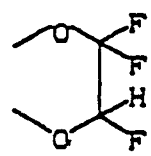

sous réserve que $R^3$ représente un groupe trifluorométhoxy lorsque $R^4$ représente le fluor, le chlore ou le brome,

et leurs sels formés par addition avec des acides et complexes de sels métalliques.

2. Dérivé 1,4-disubstitué du 1-azolyl-3,3-diméthylbutane selon la revendication 1, caractérisé par la formule

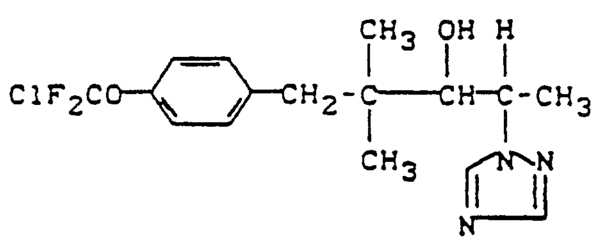 .

3. Dérivé 1,4-disubstitué du 1-azolyl-3,3-diméthylbutane selon la revendication 1, caractérisé par la formule

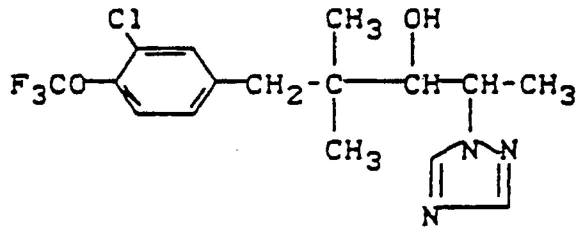 .

4. Dérivé 1,4-disubsitué du 1-azolyl-3,3-diméthylbutane selon la revendication 1, caractérisé par la formule

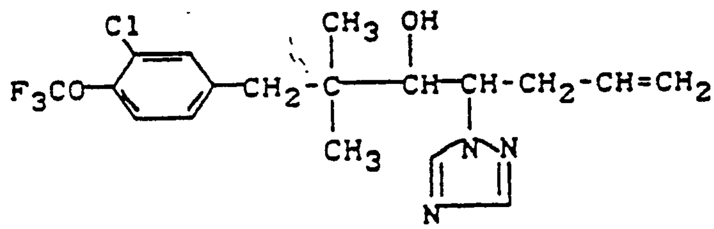 .

5. Procédé de préparation des dérivés 1,4-disubstitués du 1-azolyl-3,3-diméthylbutane de formule

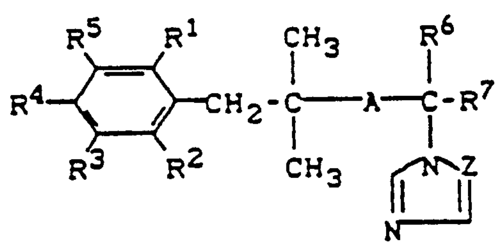 (I)

dans laquelle $R^1$, $R^2$, $R^3$, $R^4$, $R^5$, $R^6$, $R^7$, A et Z ont les significations indiquées ci-dessus, et de leurs sels formés par addition avec des acides et complexes de sels métalliques, caractérisé en ce que :

a) on fait réagir des halogénocétones de formule

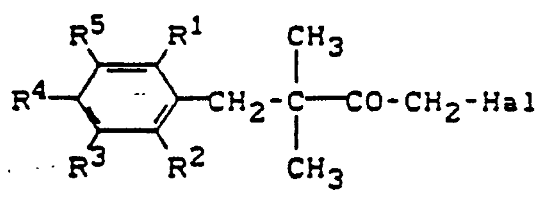 (II)

dans laquelle $R^1$, $R^2$, $R^3$, $R^4$, $R^5$ ont les significations indiquées ci-dessus, et Hal représente le chlore, le brome ou l'iode, avec un azole de formule

(III) 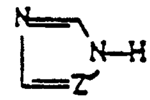

dans laquelle Z a les significations indiquées ci-dessus, en présence d'un diluant et en présence d'un accepteur d'acide, ou bien

b) on fait réagir des cétones de formule

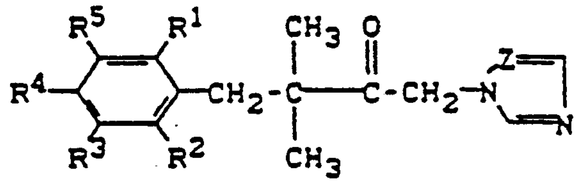 (Ia)

dans laquelle $R^1$, $R^2$, $R^3$, $R^4$, $R^5$ et Z ont les significations indiquées ci-dessus, avec un composé de formule

$R^8$ - Y (IV)

dans laquelle

$R^8$ représente un groupe alkyle à chaîne droite ou ramifiée en $C_1$-$C_{12}$ ; un groupe alcényle ou alcynyle à chaîne droite ou ramifiée contenant chacun 3 à 12 atomes de carbone;

un groupe cycloalkyle en $C_3$-$C_7$ portant éventuellement un ou deux substituants identiques ou différents choisis parmi les halogènes et les groupes alkyle; un groupe cycloalkylalkyle contenant 3 à 7 atomes de carbone dans la partie cycloalkyle et 1 à 4 atomes de carbone dans la partie alkyle et portant éventuellement un ou deux substituants identiques ou différents choisis parmi les halogènes et les groupes alkyle, ou un groupe phénylalkyle éventuellement substitué contenant 1 à 4 atomes de carbone dans la partie alkyle, les substituants du noyau phényle étant des halogènes des groupes alkyle en $C_1$-$C_4$; un groupe alcoxy ou alkylthio contenant chacun 1 à 4 atomes de carbone; un groupe halogénoalkyle, halogénoalcoxy, halogénoalkylthio, halogénoalkylsulfinyle ou halogénoalkylsulfonyle contenant chacun 1 à 4 atomes de carbone et 1 à 5 atomes d'halogènes identiques ou différents, par exemple des atomes de fluor, de chlore et/ou de brome ; un groupe nitro, cyano, alcoxycarbonyle contenant 1 à 4 atomes de carbone dans la partie alcoxy ou un groupe phényle ou phénoxy portant éventuellement un à trois substituants identiques ou différents choisis parmi le fluor, le chlore et/ou le brome, ou bien deux substituants phényle voisins forment ensemble un groupement de formule

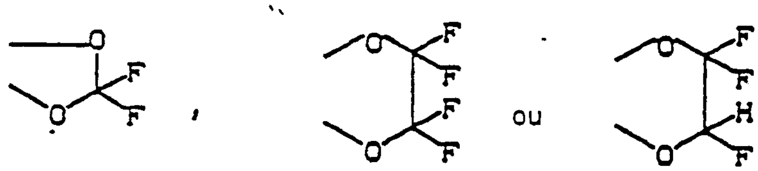

et Y représente le chlore, le brome, l'iode, un groupe p-méthylphénylsulfonyloxy, un groupement -O-$SO_2$-OR ou $NR_3$ dans lequel R représente un groupe méthyle, éthyle, n- ou iso-propyle, éventuellement en présence d'un accepteur d'acide et en présence d'un diluant et éventuellement en présence d'un catalyseur,

ou bien

c) on fait réagir des cétones de formule

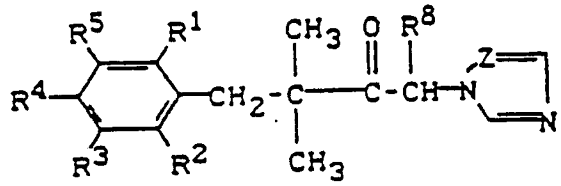

(Ib)

dans laquelle $R^1$, $R^2$, $R^3$, $R^4$, $R^5$, $R^8$ et Z ont les significations indiquées ci-dessus avec un composé de formule

$R^9$ - $Y^1$ (V)

dans laquelle
$R^9$ représente un groupe alkyle à chaîne droite ou ramifiée en $C_1$-$C_{12}$; un groupe alcényle ou alcynyle à chaîne droite ou ramifiée contenant chacun 3 à 12 atomes de carbone;
un groupe cycloalkyle en $C_3$-$C_7$ portant éventuellement un ou deux substituants identiques ou différents choisis parmi les halogènes et les groupes alkyle; un groupe cycloalkylalkyle contenant 3 à 7 atomes de carbone dans la partie cycloalkyle et 1 à 4 atomes de carbone dans la partie alkyle et portant éventuellement un ou deux substituants identiques ou différents choisis parmi les halogènes et les groupes alkyle, ou un groupe phénylalkyle éventuellement substitué contenant 1 à 4 atomes de carbone dans la partie alkyle, les substituants du noyau phényle étant des halogènes et des groupes alkyle en $C_1$-$C_4$; un groupe alcoxy ou alkylthio contenant chacun 1 à 4 atomes de carbone; un groupe halogénoalkyle, halogénoalcoxy, halogénoalkylthio, halogénoalkylsulfinyle ou halogénoalkylsulfonyle contenant chacun 1 à 4 atomes de carbone et 1 à 5 atomes d'halogènes identiques ou différents, par exemple des atomes de fluor, de chlore et/ou de brome; un groupe nitro, cyano, alcoxycarbonyle contenant 1 à 4 atomes de carbone dans la partie alcoxy ou un groupe phényle ou phénoxy portant éventuellement un à trois substituants identiques ou différents choisis parmi le fluor, le chlore et/ou le brome, ou bien deux substituants phényle voisins forment ensemble un groupement de formule

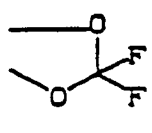 , 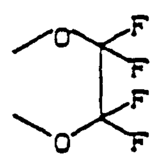 ou 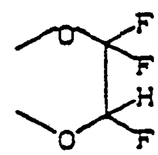

et
$Y^1$ représente le chlore, le brome, l'iode, un groupe p-méthylphénylsulfonyloxy, un groupement -O-$SO_2OR$ ou $NR_3$ dans lequel R représente un groupe méthyle, éthyle, n- ou iso-propyle,
éventuellement en présence d'un accepteur d'acide et en présence d'un diluant et éventuellement en présence d'un catalyseur,
ou bien
d) on fait réagir des cétones de formule

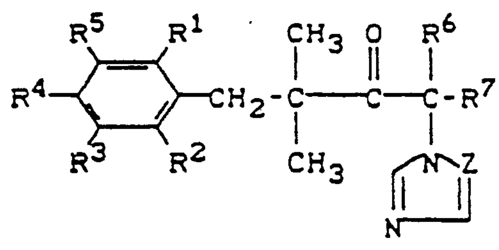

(Ic)

dans laquelle $R^1$, $R^2$, $R^3$, $R^4$, $R^5$, $R^6$ et $R^7$ ont les significations indiquées ci-dessus avec un agent réducteur, éventuellement en présence d'un diluant,

ou bien
e) on fait réagir les dérivés 1,4-disubstitués du 1-azolyl-3,3-diméthylbutane de formule

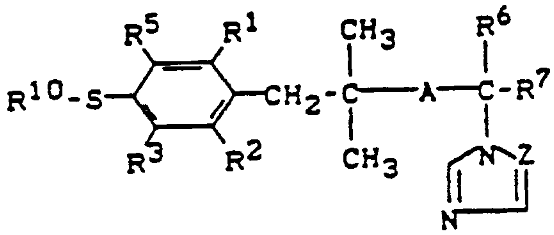

(Id)

dans laquelle $R^1$, $R^2$, $R^3$, $R^5$, $R^6$; $R^7$, A et Z ont les significations indiquées ci-dessus, et $R^{10}$ représente un groupe trifluorométhyle, difluorométhyle, trifluorochloréthyle, tétrafluoréthyle, difluorochlorométhyle ou difluorobromométhyle,
avec un noyau oxydant, éventuellement en présence d'un diluant et éventuellement en présence d'un produit auxiliaire de réaction, et le cas échéant, sur les composés de formule I ainsi obtenus, on fixe par addition un acide ou un sel métallique.

**6.** Produit fongicide, caractérisé en ce qu'il contient au moins un dérivé 1,4-disubstitué du 1-azolyl-3,3-diméthylbutane de formule I de la revendication 1 ou un sel formé par addition avec un acide ou un complexe de sel métallique d'un dérivé 1,4-disubstitué du 1-azolyl-3,3-diméthylbutane de formule I.

**7.** Utilisation des dérivés 1,4-disubstitués du 1-azolyl-3,3-diméthylbutane de formule I de la revendication 1, ou de leurs sels formés par addition avec des acides ou complexes de sels métalliques, pour la lutte contre les mycètes.

**8.** Procédé pour combattre les mycètes, caractérisé en ce que l'on applique des dérivés 1,4-disubstitués du 1-azolyl-3,3-diméthylbutane de formule I de la revendication 1 ou leurs sels formés par addition avec des acides ou complexes de sels métalliques sur les mycètes et/ou leur biotope.

**9.** Procédé de préparation de produits fongicides, caractérisé en ce que l'on mélange des dérivés 1,4-disubstitués du 1-azolyl-3,3-diméthylbutane de formule I de la revendication 1 ou leurs sels formés par addition avec des acides ou complexes de sels métalliques avec des diluants et/ou des agents tensioactifs.